# EUROPEAN PATENT APPLICATION

(11) **EP 3 670 673 A1**
(43) Date of publication of application: **24.06.2020**
(21) Application number: 18214425.3
(22) Date of filing: 20.12.2018
(51) Int. Cl.: C12Q 1/6886

(54) **IMPROVED METHODS FOR DIAGNOSIS OF LYNCH SYNDROME**

(71) Applicant: MGZ Tech GmbH, 80335 München (DE)
(72) Inventor: HOLINSKI-FEDER, Elke, 80335 München (DE); MORAK, Monika, 80335 München (DE)
(74) Representative: Bösl, Raphael Konrad

(57) **Abstract**

The present invention relates to an *in vitro* method for diagnosing Lynch Syndrome, or for determining the pathogenicity of DNA mismatch repair (MMR) gene variants, or for determining the mechanism of pathogenic DNA mismatch repair (MMR) gene defects or Lynch Syndrome, in a patient suffering from Lynch Syndrome, suspected to suffer from Lynch Syndrome or having a risk to develop Lynch Syndrome, the method comprising: (ai) determining for full-length transcripts (FLTs) of the DNA mismatch repair (MMR) genes MLH1, MSH2, MSH6 and PMS2 in a sample comprising cells obtained from the patient: the sequence of the FLTs, thereby determining the degree of splicing of the exon(s) and the protein sequence encoded by the FLTs, and (aii) determining allelic balance of a MMR gene of (ai).

SEQUENCE LISTING

<110>  MGZ Tech GmbH

<120>  Improved methods for diagnosis of Lynch Syndrome

<130>  M72052EP

<160>  10

<170>  PatentIn version 3.5

<210>  1
<211>  18
<212>  DNA
<213>  Artificial Sequence

<220>
<223>  Oligo-(dT)18 primer

<400>  1
tttttttttt tttttttt                                                    18


<210>  2
<211>  20
<212>  DNA
<213>  Artificial Sequence

<220>
<223>  MLH1 transcript forward primer

<400>  2
tgtccaatca atagctgccg                                                  20


<210>  3
<211>  19
<212>  DNA
<213>  Artificial Sequence

<220>
<223>  MLH1 transcript reverse primer

<400>  3
agaacacatc ccacagtgc                                                   19


<210>  4
<211>  20
<212>  DNA
<213>  Artificial Sequence

<220>
<223>  MSH2 transcript forward primer

<400>  4
gtcgcgcatt ttcttcaacc                                                  20


<210>  5
<211>  19
<212>  DNA

<213>  Artificial Sequence

<220>
<223>  MSH2 transcript reverse primer

<400>  5
aagttgatag cccatgggc                                                    19


<210>  6
<211>  19
<212>  DNA
<213>  Artificial Sequence

<220>
<223>  MSH6 transcript forward primer

<400>  6
cgtccgacag aacggttgg                                                    19


<210>  7
<211>  21
<212>  DNA
<213>  Artificial Sequence

<220>
<223>  MSH6 transcript reverse primer

<400>  7
ccacctttgt cagaagtcaa c                                                 21


<210>  8
<211>  22
<212>  DNA
<213>  Artificial Sequence

<220>
<223>  PMS2 transcript forward primer

<400>  8
gttgcatcca tggagcgagc tg                                                22


<210>  9
<211>  21
<212>  DNA
<213>  Artificial Sequence

<220>
<223>  PMS2 transcript reverse primer

<400>  9
ttgcaagcaa tgctccatct g                                                 21


<210>  10
<211>  71
<212>  DNA
<213>  Homo sapiens

<400>  10

aatgtgccat gtgaaccacc ccgtctgaaa agtgaggagc ccctctgccc ggcagccgcc 60

ccgtctggga g 71

## Description

The present invention relates to an *in vitro* method for diagnosing Lynch Syndrome, or for determining the pathogenicity of DNA mismatch repair (MMR) gene variants, or for determining the mechanism of pathogenic DNA mismatch repair (MMR) gene defects or Lynch Syndrome, in a patient suffering from Lynch Syndrome, suspected to suffer from Lynch Syndrome or having a risk to develop Lynch Syndrome, the method comprising: (ai) determining for full-length transcripts (FLTs) of the DNA mismatch repair (MMR) genes *MLH1, MSH2, MSH6* and *PMS2* in a sample comprising cells obtained from the patient: the sequence of the FLTs, thereby determining the degree of splicing of the exon(s) and the protein sequence encoded by the FLTs, and (aii) determining allelic balance of a MMR gene of (ai).

Heterozygous germline defects in the four major DNA mismatch repair (MMR) genes *MLH1, MSH2, MSH6,* and *PMS2* represent the genetic cause for Lynch Syndrome (LS), the most common hereditary predisposition for colorectal cancer (CRC) and associated tumors following an autosomal dominant mode of inheritance (Lynch and de la Chapelle, 1999).

LS associated tumors typically show high microsatellite instability (MSI-H) and an immunohistochemical (IHC) loss of the respective MMR protein expression. For 53-65% of the patients documented in the German HNPCC Consortium and fulfilling the clinical criteria of LS, a pathogenic germline variant in a MMR gene confirms the diagnosis (Mangold, et al., 2005; Steinke, et al., 2013), assigned as "class 5" by the InSiGHT Mismatch Repair Gene Variant Classification Criteria in the LOVD (Thompson, et al., 2014). However, the detection of either a germline variant of uncertain significance (VUS) (>20%), or no relevant variant at all (>15%) in the MMR genes leaves one third of these patients unsolved (Kayser, et al., 2018; Mangold, et al., 2005; Muller-Koch, et al., 2001; Peltomaki and Vasen, 2004). The accumulation of two somatic mutations may explain several patients, but not those with familial tumor clustering (Mensenkamp, et al., 2014; Vargas-Parra, et al., 2017). Amongst VUS, missense variants can exert a functional defect on protein level, for which *in silico* prediction programs and *in vitro* assays are performed to define their pathogenicity (Andersen, et al., 2012; Borras, et al., 2012; Drost, et al., 2012; Hardt, et al., 2011; Ollila, et al., 2006), but they can also confer a splicing defect.

The identification of splicing defects as a primary cause of disease implies the possibility of therapeutic approaches that target splicing (Cartegni and Krainer, 2003; Soret, et al., 2006). Therefore, a correct assessment of the pathogenicity mechanism of a given germline variant is mandatory. Splice prediction programs are very reliable only for variants within the conserved splice sites (Auclair, et al., 2006; Lastella, et al., 2006), but not for variants altering an exonic splice enhancer (ESE) or generating an exonic splice silencer (ESS) as well as intronic motifs. Furthermore, the *in vivo* effect of SSD variants can be versatile and incomputable *in silico* (Gerykova-Bujalkova, et al., 2008). There is also growing evidence that many supposed missense variants in the MMR genes result in fact in a splicing defect (Auclair, et al., 2006; Gerykova-Bujalkova, et al., 2008; van der Klift, et al., 2015). The same is probably true for assumed synonymous variants and small indels, even though to a lower amount (Auclair, et al., 2006; van der Klift, et al., 2015; Yamaguchi, et al., 2017), and even for truncating variants (Baehring, et al., 2006; Nystrom-Lahti, et al., 1999; Stella, et al., 2001). For a correct pathogenicity assessment of sequence variants, a splicing defect should be excluded prior to functional analyses on protein level. Therefore, cDNA analyses are necessary to investigate the effect of variants on mRNA level. This, however, is impeded by the presence of alternatively spliced isoforms of the MMR genes which were already reported frequently in literature (Charbonnier, et al., 1995; Clarke, et al., 2000; Genuardi, et al., 1998; Takahashi and Nagai, 2009). The diverse alternative isoforms of the MMR genes are regarded as non-functional (Nystrom-Lahti, et al., 1999; Thompson, et al., 2015) and therefore benign, but have to be discriminated from an aberrant, allele-specific splicing (Spurdle, et al., 2008; Vreeswijk and van der Klift, 2012). This implies that exon skipping in MMR transcripts exceeding a certain threshold has to be considered as aberrant splicing and pathogenic, even for exons known to be alternatively spliced and for isoforms with if deletions. The detection of aberrant splicing in presence of a VUS helps in pathogenicity assessment of that variant, especially when a complete, allele-specific SSD can be demonstrated.

Accordingly, heterozygous germline defects in the DNA mismatch repair (MMR) genes *MLH1, MSH2, MSH6,* or *PMS2* predispose to Lynch Syndrome (LS). However, in 35-47% of the patients fulfilling the clinical criteria of LS, either a VUS or no causative variant is detected, so that tumor risk and heritability remain indefinite.

Accordingly, there is a need for improved methods for diagnosing Lynch Syndrome, or for determining the pathogenicity of DNA mismatch repair (MMR) gene variants, or for determining the mechanism of pathogenic DNA mismatch repair (MMR) gene defects or Lynch Syndrome, in a patient suffering from Lynch Syndrome, suspected to suffer from Lynch Syndrome or having a risk to develop Lynch Syndrome.

The problem is solved by the methods of the present invention.

The present invention relates to an *in vitro* method for diagnosing Lynch Syndrome, or for determining the pathogenicity of DNA mismatch repair (MMR) gene variants, or for determining the mechanism of pathogenic DNA mismatch repair (MMR) gene defects or Lynch Syndrome, in a patient suffering from Lynch Syndrome, suspected to suffer from Lynch Syndrome or having a risk to develop Lynch Syndrome, the method comprising: (ai) determining for full-length transcripts (FLTs) of the DNA mismatch repair (MMR) genes MLH1, MSH2, MSH6 and PMS2 in a sample comprising cells obtained from the patient: the sequence of the FLTs, thereby determining the degree of splicing of the exon(s) and the protein sequence encoded by the FLTs, and (aii) determining allelic balance of a MMR gene of (ai).

In particular, it was surprisingly found that the analysis of full-length transcripts of all four MMR genes *MLH1, MSH2, MSH6* and *PMS2,* including their sequences and the allelic balance in a genomically heterozygous variant of these MMR genes results in an improved method for diagnosing Lynch Syndrome.

In particular, we set-up a cDNA analysis for full-length transcripts (FLTs) of these four MMR genes using patients' lymphocyte cultures with active and inhibited nonsense-mediated mRNA decay (NMD) to analyze the splicing pattern and transcript integrity. To define cut off levels for alternative and aberrant splicing, the method was validated on ten normal controls, 14 samples with variants generating an early or late premature termination codon (PTC), six samples with variants classified as splice site defect (SSD), and six samples with pathogenic putative missense variants. Then the FLT analysis was applied to 21 patients harbouring a VUS and 26 patients with unexplained immunohistochemical MMR protein loss in their tumor. 15% of all putative missense variants turned out to be SSDs, and in 19% of the hitherto unsolved patients an allelic loss or aberrant transcript was detected. Our method enhances diagnostic accuracy by finding variants/defects and assessing the mechanism of pathogenicity of MMR gene variants and particularly VUSs, which gains relevance in the context of precision medicine and gene therapy, and helps optimizing prediction programs. The valuable experience and data gained will be applied for calibrating a high-throughput NGS-based mRNA analysis.

In more detail, for pathogenicity assessment of variants on mRNA-level, we established a robust and universal protocol for cDNA analysis of the four MMR FLTs by leukocyte culture, investigation of active and inhibited NMD in cDNA without and with puromycin incubation (designated "cDNA-P"/"cDNA+P"), RT-PCR and sequencing, thereby defining the normal alternative splicing pattern, and the cut off value for aberrant splicing. Finally, the system was tested on samples with assumed splice site variants, truncating and pathogenic variants, VUSs, and on samples from molecularly unsolved patients suspected of having LS.

The determination of the values in (ai) and (aii) is preferably performed semi-quantitatively or quantitatively.

Hereditary nonpolyposis colorectal cancer (HNPCC) or Lynch syndrome (LS) is an autosomal dominant genetic condition that has a high risk of colon cancer as well as other cancers including endometrium (second most common), ovary, stomach, small intestine, hepatobiliary tract, upper urinary tract, brain, and skin. The increased risk for these cancers is due to inherited mutations that impair DNA mismatch repair. It is a type of hereditary cancer syndrome.

Patients which belong to a family wherein said family history includes diagnoses of colorectal and/or endometrial cancer in multiple relatives on the same side of a family are particularly suspected to suffer from Lynch Syndrome or suspected of having a risk to develop Lynch Syndrome. In addition, cancers associated with Lynch syndrome are more likely to be diagnosed at a young age. People with Lynch syndrome are also at an increased risk of developing multiple types of cancers during their lifetime. Accordingly, patients developing a cancer early in life or multiple cancers are further preferred patients suspected to suffer from Lynch Syndrome or having a risk to develop Lynch Syndrome. Further preferred patients suspected to suffer from Lynch Syndrome or having a risk to develop Lynch Syndrome are patients having at least one relative which is diagnosed to suffer from Lynch Syndrome.

Heterozygous germline defects in the DNA mismatch repair (MMR) genes *MLH1, MSH2, MSH6,* or *PMS2* predispose to Lynch Syndrome (LS). It is known that different germline defects exhibit different degree of pathogenicity. Pathogenicity is well accepted to be classified pursuant to the InSiGHT classification. For the present invention, the InSiGHT Variant Interpretation Committee MMR gene variant classification criteria (Version 2.4 June 2018) and the corresponding database LOVD3 apply.

The pathogenicity of variants is typically classified according to the established InSiGHT criteria as class 1 (benign), class 2 (likely benign), class 3 (Variant of Uncertain Significance, VUS), class 4 (likely pathogenic), and class 5 (pathogenic).

The methods of the present invention allows for determining the pathogenicity of MMR gene variants, by investigating their effect upon mRNA splicing, determining the mechanism of pathogenic DNA mismatch repair (MMR) gene defects and/or for determining the mechanism of Lynch Syndrome in a subject.

The patient or subject to be analyzed is a mammal, preferably a human.

The full-length transcripts or FLTs are from a sample comprising cells obtained from the patient.

Typically, RNA comprising mRNA, such as total RNA or mRNA is enriched or isolated from the sample comprising cells, using methods known in the art. For example, the cells may be lysed or partially lysed. Further, DNA may be digested and/or separated from RNA. An RNAse inhibitor may be added to the sample and/or cells.

Suitable samples encompass a blood sample, in particular whole blood sample, or a tissue sample, in particular sample comprising colon mucosa, or sputum. The sample obtained from the patient comprises cells expressing MMR mRNA. In the examples, lymphocytes were used, as these cells can be obtained easily and in sufficient amount from a blood sample of the patient. It is, however, possible to also use other cells. Therefore, the sample preferably comprises lymphocytes, in particular Peripheral Blood Lymphocytes (PBL). Blood or sputum samples may be obtained by methods known in the art. For a sample comprising colon mucosa, a biopsy may be taken.

In one preferred embodiment, said sample is a blood sample, in particular whole blood sample, or a tissue sample, in particular sample comprising colon mucosa, or sputum.

In one preferred embodiment, the cells are lymphocytes, in particular Peripheral Blood Lymphocytes (PBL).

The sample may be stored at 4°C or less, or frozen, such as at temperatures of - 4°C or less, -10°C or less, or -20°C or less, until use.

The cells in the samples, in particular the lymphocytes, are preferably cultured in a suitable medium at about 37°C, for expressing MMR transcripts. For example, the cells are cultured for about 2 days to 10 or 20 days. In the examples, the cells were cultured for 72 h to 96 h at 37°C, and incubated for 5 h at 37°C in parallel approaches without (designated cDNA-P) and with (designated cDNA+P) puromycin, respectively, according to the common general knowledge described in Andreutti-Zaugg, et al., 1997 and Vreeswijk and van der Klift, 2012. Without the addition of puromycin, the nonsense-mediated mRNA decay (NMD) pathway is active in the cells. With the addition of puromycin, the nonsense-mediated mRNA decay (NMD) pathway is inactive in the cells.

Nonsense-mediated mRNA decay (NMD) in mammalian cells is a mechanism for the removal of mRNA containing premature stop codons and is mediated by the coordinated function of numerous proteins that dynamically associate with the exon junction complex.

In the method of the invention, the full-length transcripts (FLTs) of the DNA mismatch repair (MMR) genes *MLH1, MSH2, MSH6* and *PMS2* are analyzed.

Human *MLH1, MSH2, MSH6* and *PMS2* genes and sequences of full-length transcripts as well as sequences of the encoded proteins are known to a skilled person. In particular, NM_000249.3 (version of 18-Nov-2018), NG_007109.2 (version of 12-NOV-2018) are known for *MLH1,* NM_000251.2 (version of 23-OCT-2018), NG_007110.2 (version of 16-SEP-2018) are known for *MSH2,* NM_000179.2 (version of 10-NOV-2018), NG_007111.1 (version of 12-SEP-2018) are known for *MSH6,* and NM_000535.5 (version of 13-JUL-2015), NP_000526.2 (version of 18-NOV-2018) are known for *PMS2.*

*MLH1* is also known as mutL homolog 1.

*MSH2* is also known as mutS homolog 2.

*MSH6* is also known as mutS homolog 6.

*PMS2* is also known as PMS1 homolog 2, mismatch repair system component.

In order to determine allelic balance for a MMR gene, and determining for full-length transcripts (FLTs) of the DNA mismatch repair (MMR) genes *MLH1, MSH2, MSH6* and *PMS2* in a sample comprising cells obtained from the patient: the sequence of the FLTs, thereby determining the degree of splicing of the exon(s) and the protein sequence encoded by the FLTs, pursuant to steps (ai) and (aii) of the present invention, various methods may be used. For example, the mRNA may be analyzed directly, or may be reverse transcribed into cDNA and be analyzed subsequently. In case mRNA is analyzed directly, the mRNA sequence may be determined by sequencing using RNA sequencing methods known in the art. For example, the Illumina sequencing by synthesis (SBS) chemistry may be used for mRNA sequencing.

A full-length transcript or FLT is understood as an mRNA molecule transcribed *in vivo* in the cells of the patient, wherein the RNA molecule comprises all exons of said mRNA molecule comprising at least the start methionine codon and the usual stop codon, and at least 50%, 60%, 70%, 80%, 90%, 95%, 98%, 99% or 100% of the 5' untranslated region (UTR), and at least 50%, 60%, 70%, 80%, 90%, 95%, 98%, 99% or 100% of the 3'UTR of said mRNA molecule, in particular until the first possible polyadenylation site. In the present invention, the term full-length transcript or FLT also refers to a cDNA molecule derived therefrom or a nucleic acid molecule having a sequence complementary or reverse complementary thereto. Accordingly, the full-length transcript or FLT may be single-stranded or double-stranded. The full-length transcript or FLT may be an RNA or DNA. In case the FLT is an mRNA, the FLT is preferably single-stranded. In case the FLT is a cDNA, the FLT is single-stranded or double-stranded.

In a preferred embodiment, full-length cDNA is prepared from full-length mRNA transcripts of the MMR genes. Methods for preparing cDNA from mRNA are well-known in the art and in particular include reverse transcription using a reverse transcriptase.

With a cDNA analysis of a MMR transcript and demonstration of biallelic expression defects within the transcript analyzed can be excluded (but not before the forward primer and not behind the reverse primer employed).

With a normal cDNA result for the *MSH2* transcript, to exclude Lynch Syndrome, optionally, a germline deletion screening of *EPCAM* exon 9 including its stop codon could in addition be performed, in order to exclude a tissue-specific silencing of *MSH2.*

In a particularly preferred embodiment, Long-Range Reverse-Transcriptase Polymerase Chain Reaction is performed, in order to prepare full-length cDNA from full-length RNA transcripts.

Long-Range Reverse-Transcriptase Polymerase Chain Reaction, or Long Range RT-PCR or LR-PCR encompasses Long-Range reverse transcription, followed by Long Range PCR.

Long-Range reverse transcription is understood as method for reverse transcription of mRNA lengthes that cannot typically be reverse transcribed using routine reverse transcription methods or reagents. For typical mRNA templates, polymerases optimized for Long-Range reverse transcription are capable of reverse transcribing at least 3 kb, 4 kb or 5 kb and for example up to 10 kb, 20 kb, 30 kb or even beyond. Such methods employing suitable reverse transcriptases are known in the prior art.
Long-Range PCR is understood as method for amplification of DNA lengthes that cannot typically be amplified using routine PCR methods or reagents. For typical DNA templates, polymerases optimized for Long-Range PCR are capable of amplifying at least 3 kb, 4 kb or 5 kb and for example up to 10 kb, 20 kb, 30 kb or even beyond. Such methods employing suitable polymerases are known in the prior art. For example, polymerases and methods in the Examples may be used. Long-Range RT-PCR or LR-PCR is understood as method for reverse transcription and amplification of DNA lengthes that cannot typically be amplified using routine PCR methods or reagents. For typical DNA templates, polymerases optimized for Long-Range PCR are capable of amplifying at least 3 kb, 4 kb or 5 kb and for example up to 10 kb, 20 kb, 30 kb or even beyond. Such methods employing suitable polymerases are known in the prior art. For example, polymerases and methods in the Examples may be used.

For amplification of full-length transcripts, it is preferred to use, for each MMR, at least one forward primer located in the 5' untranslated region (UTR) of the first exon, and at least one reverse primer located in the 3'UTR of the last exon of the longest transcript.

According to (aii) of the method of the invention, allelic balance is determined. Allelic balance is understood as the percentage value of amount of a certain allele as compared to the second allele. For determining allelic balance, a known genomically heterozygous MMR gene variant is typically selected as "informative variant" for determining allelic balance. Therefore, in one preferred embodiment of the invention, allelic balance is determined in the presence of a genomically heterozygous variant of a MMR gene and/or is determined in at least one genomically heterozygous variant of a MMR gene. For said informative variant, allelic balance can be determined e.g. as described in the examples, by determining the relative amounts of the alleles, expressed as %-value. In a preferred embodiment, the relative amounts of the alleles are determined e.g. by determining, for each allele, the signal intensities obtained from sequences of the FLTs. The relative amounts of signal intensities may be e.g. determined by visual inspection of the amount of respective FLT sequences, by eye, or by computational data processing of the signal intensities. Accordingly, the analysis may be performed quantitatively or semi-quantitatively. Alternative methods for determining allelic methods are known in the art and include quantitatively determining the amount of the alleles, e.g. by suitable sequencing methods, such as nanopore sequencing.

Preferably, as in the examples, the sequencing peak with higher intensity in a heterozygous variant is defined as one allele representing 50%, and the sequencing peak with lower intensity is calculated accordingly.

When determining allelic balance, the value for the amount or signal intensity of the allelic balance for a MMR gene is understood as "biallelic" in case the value for allelic balance is between 40% and 60%.

In one preferred embodiment, a value for allelic balance described herein refers to the value for allelic balance in the presence of a genomically heterozygous variant of a MMR gene and/or in at least one genomically heterozygous variant of a MMR gene.

In case of a biallelic expression, the amount and/or intensities of the wildtype FLT and alternatively/aberrantly spliced isoforms are set to represent 100% in sum.

When determining allelic balance, the value for the amount or signal intensity of the allelic balance for a MMR gene is determined as "monoallelic expression" or as "allelic loss", in case the value for one allele the amount and/or intensity is between 0% and 10%.

According to step (ai) of the method of the invention, for full-length transcripts (FLTs) of the DNA mismatch repair (MMR) genes *MLH1, MSH2, MSH6* and *PMS2* in a sample comprising cells obtained from the patient, the sequence of the FLTs is determined, thereby determining the degree of splicing of the exon(s) and the protein sequence encoded by the FLTs.

Various aberrantly spliced isoforms, encompassing, for example, variants inducing exon skipping in-frame (*if*) or out of frame (*oof*), generating premature termination variants (PTC), large genomic deletions/duplications, inversions, large genomic insertions, and intronic variants, such as deep intronic variants, and variants at the intronic/exonic boundary, are known for the MMR genes.

Sequencing methods in the art may be used for determining the sequence of the FLTs. In the example, Sanger sequencing is used. However, any other sequencing method, such as Next Generation Sequencing methods, may be used. For example, emulsion PCR may be used. Emulsion PCR encompasses isolating individual DNA molecules along with primer-coated beads in aqueous droplets within an oil phase. A polymerase chain reaction (PCR) then coats each bead with clonal copies of the DNA molecule followed by immobilization for later sequencing. Suitable sequencing methods known in the art include, for example, single-molecule real-time sequencing (Pacific Biosciences), pyrosequencing (454), sequencing by synthesis (Illumina), combinatorial probe anchor synthesis (cPAS-BGI/MGI), sequencing by ligation (SOLiD sequencing), Nanopore Sequencing, and chain termination sequencing (Sanger sequencing).

For MMR genes, splice variants or isoforms exceeding a certain threshold of usual alternative splicing are considered as aberrant splicing and pathogenic, even for exons known to be alternatively spliced and for isoforms with if deletions. In the methods of the present invention, cut-off values are determined for "alternative splicing" and "aberrant splicing", respectively.

Typically, unless otherwise specified for specific exon-specific splice variants tolerating higher values of alternative splicing, "aberrant splicing" is understood as a degree of splicing for a MMR gene which is between 30% and 50% for the exon(s) of said MMR gene. Accordingly, a degree of splicing which is below the cut off value for aberrant splicing of 30% and above the values defined for "alternative splicing" may be considered of unclear significance or pathogenic if combined with an allelic reduction.

In a preferred embodiment, the relative amounts of the alternatively spliced isoforms are determined by determining, for each isoform for a MMR, the signal intensities obtained from sequencing the FLTs. The relative amounts of signal intensities may be e.g. determined by visual inspection of the amount of respective FLT sequences, by eye, or by computational data processing of the signal intensities. Accordingly, the analysis may be performed quantitatively or semi-quantitatively.

By determining the protein sequence encoded by the FLTs, effects of the splice variant on the encoded protein sequence can be determined, e.g. exon skipping resulting in in frame (if) isoforms, out of frame (*oof*) isoforms or isoforms generating premature termination variants (PTC) can be identified. Further, variants can be predicted to be pathogenic or likely pathogenic pursuant to the InSiGHT classification, e.g. by identifying early premature termination variants (PTC) or large inversions, insertions and/or deletions.

It was surprisingly found that Lynch Syndrome is diagnosed or a DNA mismatch repair (MMR) gene variant is determined to be pathogenic or likely pathogenic, or not to be splice-neutral to impair normal splicing, or to constitute a splicing defect, in case the allelic balance for a MMR gene is monoallelic with a value of between 0% and 10% . The values are considered as allelic loss pursuant to the present invention.

Accordingly, an allelic loss is preferably found to be pathogenic or likely pathogenic.

It was surprisingly found that Lynch Syndrome is diagnosed or a DNA mismatch repair (MMR) gene variant is determined to be pathogenic or likely pathogenic, or not to be splice-neutral, in case the allelic balance for a MMR gene is biallelic with a value of between 40% and 60% in a genomically heterozygous variant, which is not the splicing variant, and the degree of splicing is between 30% and 50% for the exon(s) of said MMR gene, which is understood as aberrant splicing pursuant to the present invention.

Accordingly, even in case of biallelic expression, a MMR gene variant is determined to be pathogenic or likely pathogenic, or not to be splice-neutral, in case the allelic balance for a MMR gene is biallelic with a value of between 40% and 60%, if the degree of splicing is between 30% and 50% for the exon(s) of said MMR gene, representing aberrant splicing of said MMR gene.

It was further surprisingly found that Lynch Syndrome is diagnosed or a DNA mismatch repair (MMR) variant is determined to be pathogenic or likely pathogenic, or not to be splice-neutral, in case the allelic balance for a MMR gene is biallelic with a value of between 40% and 60% in a genomically heterozygous variant, wherein the protein sequence encoded by the FLT of said variant is predicted to be pathogenic or likely pathogenic (InSiGHT class 5 or 4).

Accordingly, even in case of biallelic expression, a MMR gene variant is determined to be pathogenic or likely pathogenic, or not to be splice-neutral, in case the allelic balance for a MMR gene is biallelic with a value of between 40% and 60%, if the protein sequence encoded by the FLT of said variant is predicted to be pathogenic or likely pathogenic (InSiGHT class 5 or 4), e.g. by identifying a early premature termination variant (PTC) or large inversion, insertion and/or deletion.

It was further found that the patient is determined not to suffer from Lynch syndrome due to a germline MMR-defect, or a DNA mismatch repair (MMR) gene variant is determined to be splice-neutral, in case:
the allelic balance for the respective MMR gene is biallelic with a value of between 40% and 60%, wherein: i) the protein sequence encoded by the FLT of said variant is predicted to be benign or likely benign (InSiGHT class 1-2) or ii) said variant is a synonymous variant or intronic variant, and
the degree of splicing is between 0% and 10% for said MMR exons,
with the proviso of exon-specific splice variants of a MMR gene are tolerated with following values for the degree of splicing between 0% and 15% for skipping of *MLH1* exon 1q, *PMS2* exon 6p and exon 11, and between 0% and 25% for *MSH6* exon 4, which is understood as alternative splicing.

The protein sequence encoded by the FLT of said variant is predicted to be benign or likely benign (InSiGHT class 1-2), in case the predicted protein sequence exhibits only comparably minor alterations of the encoded protein sequence as compared to the wildtype variant, such as e.g. deletions or insertions in frame of less 50, 40, 30, 20, 10 or 5 amino acids, in particular wherein the deletions or insertions in frame are located outside a functional domain of the MMR protein.

The protein sequence encoded by the FLT of said variant is predicted to be pathogenic or likely pathogenic (InSiGHT class 5 or 4), in case the predicted protein sequence exhibits major alterations of the encoded protein sequence as compared to the wildtype variant, such as e.g. deletions or insertions of more than 50, 60, 70, 80, 90 or 100 amino acids, e.g. by generating a early premature termination variant (PTC), a large insertion or deletion.

For a few specific isoforms or splice variants, higher values for the degree of exon splicing were found to be tolerated. In particular, a degree of splicing of between 0% and 15% for skipping of *MLH1* exon 1q, *PMS2* exon 6p and exon 11, and between 0% and 25% for *MSH6* exon 4, is understood as "alternative splicing" pursuant to the present invention.

A synonymous variant is understood as a gene variant which does not result in alterations of the encoded protein sequence as compared to the wildtype variant.

It was further found that a DNA mismatch repair (MMR) gene variant is determined to be splice-neutral, in case the allelic balance for the respective MMR gene is biallelic with a value of between 40% and 60%, and the degree of splicing is between 0% and 10% for said MMR exons, with the proviso of exon-specific splice variants of an MMR gene are tolerated with following values for the degree of splicing between 0% and 15% for skipping of *MLH1* exon 1q, *PMS2* exon 6p and exon 11, and between 0% and 25% for *MSH6* exon 4, which is considered to represent alternative splicing.

A "splice-neutral" MMR gene variant is understood as a MMR gene variant which does not alter the splicing pattern compared to the wildtype MMR gene. A splice-neutral MMR gene variant is typically determined to be benign or likely benign on RNA-level, comprising the biallelic expression of the variant if located in the analyzed FLT.

Variants predicted to alter the amino acid sequence of the protein encoded by the MMR without disrupting the reading frame, such as a missense variant altering one or up to four amino acid(s), in-frame deletion or in-frame insertion of one to up to four amino acid(s), and small indels can be demonstrated to be splice-neutral in the examples using the methods of the invention.

As these variants have an effect on the encoded protein sequence, functional analyses on protein level can be performed in addition, in order to provide a further pathogenicity assessment.

In another preferred embodiment, variants located in the intronic region which were demonstrated to be splice-neutral are classified as InSiGHT class 1-2 (benign or likely benign).

In another preferred embodiment, variants predicted not to alter the amino acid sequence of the protein encoded by the FLT (synonymous variant) which were demonstrated to be splice-neutral are classified as InSiGHT class 1-2 (benign or likely benign).

Accordingly, independent from the predicted encoded protein sequence, MMR gene variants are considered to be splice-neutral in case of biallelic expression and a degree of splicing which is between 0% and 10% for said MMR exons, wherein higher values are tolerated for the specific MMR gene variants *MLH1* exon 1q, *PMS2* exon 6p and exon 11, and *MSH6* exon 4, as specified above.

In one preferred embodiment of methods of the invention, a variant is characterized as splicing defect when causing aberrant splicing of at least one exon or intron, in part or completely, which is located in or in close genomic proximity to the position of said splicing variant, such as located in or within a distance of 1, 2, 3, 4, 5, 6, 7, 8, 9, 10, 15 or 20 nucleotides to the position of the splice variant, or within the same exon as the variant. Alternatively, an aberrant isoform or splice variant may be a deep intronic variant or a variant of the branch site.

In a yet further preferred embodiment, variants in the promoter or in 5' or 3' UTR of the MMR gene transcript can be determined as non-pathogenic or splice-neutral or regulatory-neutral, in case transcription integrity can be proven for the respective MMR gene by an allelic balance with a value of between 40% and 60% (InSiGHT class 1-2), and
the degree of splicing is between 0% and 10% for the MMR exons, and exon-specific exceptions tolerating higher values: up to 15% is tolerated for skipping of *MLH1* exon 1q, or *PMS2* exon 6p and exon 11, and up to 25% for *MSH6* exon 4 (alternative splicing).

In a yet further preferred embodiment, variants predicted to alter the amino acid sequence without disrupting the reading frame, such as missense variants, in-frame deletion of amino acid(s) or in-frame insertion of amino acid(s), which were demonstrated to be splice-neutral, need further investigation and data regarding the functional relevance on protein level for classification.

Therefore, in a preferred embodiment, the method of the invention further comprises step (b):
(b) diagnosing Lynch Syndrome or determining the pathogenicity of DNA mismatch repair (MMR) gene variants, or determining the mechanism of pathogenic DNA mismatch repair (MMR) gene defects or Lynch Syndrome, in the patient, wherein:
   (b1) Lynch Syndrome is diagnosed or a DNA mismatch repair (MMR) gene variant is determined to be pathogenic or likely pathogenic, or not to be splice-neutral
      i) in case the allelic balance for a MMR gene is monoallelic with a value of between 0% and 10% (allelic loss), or
      ii) in case the allelic balance for a MMR gene is biallelic with a value of between 40% and 60%, and
         the degree of splicing is between 30% and 50% for the exons of said MMR gene (aberrant splicing), or
      iii) in case the allelic balance for a MMR gene is biallelic with a value of between 40% and 60%, wherein the protein sequence encoded by the FLT of said variant is predicted to be pathogenic or likely pathogenic (InSiGHT class 5 or 4), and
   (b2) the patient is determined not to suffer from Lynch syndrome due to a germline MMR gene defect, or a DNA mismatch repair (MMR) gene variant is determined to be splice-neutral, in case
      the allelic balance for the respective MMR gene is biallelic with a value of between 40% and 60%, wherein: i) the protein sequence encoded by the FLT of said variant is predicted to be benign or likely benign (InSiGHT class 1-2) or ii) said variant is a synonymous variant or intronic variant, and
      the degree of splicing is between 0% and 10% for said MMR exons,
      with the proviso of exon-specific splice variants of an MMR tolerated with following values for the degree of splicing between 0% and 15% for skipping of *MLH1* exon 1q, *PMS2* exon 6p and exon 11, and between 0% and 25% for *MSH6* exon 4 (alternative splicing),
      or
      a DNA mismatch repair (MMR) gene variant is determined to be splice-neutral, in case
      the allelic balance for the respective MMR gene is biallelic with a value of between 40% and 60%, and
      the degree of splicing is between 0% and 10% for said MMR exons,
      with the proviso of exon-specific splice variants of an MMR tolerated with following values for the degree of splicing between 0% and 15% for skipping of *MLH1* exon 1q, *PMS2* exon 6p and exon 11, and between 0% and 25% for *MSH6* exon 4 (alternative splicing).

Accordingly, cut-off values for reliably diagnosing and determining pathogenicity of LS could be established.

In case the values determined for a sample neither fulfill the criteria of (b1) nor the criteria of (b2), further investigation of the variant is required in order to classify the variant. For example, immunohistochemical analyses and/or additional analyses of mRNA splicing and stability could be performed.

It was further found that an even more accurate diagnosis is achieved if the cells in the sample are cultured and incubated in the absence and presence of puromycin. In case the cells in the sample are incubated and/or cultured without puromycin in the culturing medium conditions, the nonsense-mediated mRNA decay (NMD) pathway is active in the cells.

In the examples, it could be demonstrated that transcripts with a late premature termination (PTC) are not subjected to NMD. Transcripts with an early PTC at least 55 nucleotides prior to the last exon-exon-junction were underrepresented with 0-10% in cells incubated in the absence of puromycin (designated "cDNA-P" in the examples) due to NMD and defined as an allelic loss, but can be restored up to 40% intensity in cells incubated in the presence of puromycin (designated "cDNA+P" in the examples), resulting in NMD-inhibition.

Accordingly, in one preferred embodiment of the method of the invention, in steps (ai) and (aii), the nonsense-mediated mRNA decay (NMD) pathway is active in the cells in said sample. Therefore, in this embodiment, the cells of the sample are maintained and/or cultured in the absence of puromycin.

In a more preferred embodiment, step (b) comprises, for the alternative of an active NMD pathway:
(b) diagnosing Lynch Syndrome or determining the pathogenicity of DNA mismatch repair (MMR) variants, or determining the mechanism of pathogenic DNA mismatch repair (MMR) gene defects or Lynch Syndrome, in the patient, wherein:
   (b1) Lynch Syndrome is diagnosed or a DNA mismatch repair (MMR) gene variant is determined to be pathogenic or likely pathogenic, or not to be splice-neutral,
      i) in case the allelic balance for a MMR gene is monoallelic with a value of between 0% and 10%, or
      ii) in case the allelic balance for a MMR gene is biallelic with a value of between 40% and 60%, and
         the degree of *in-frame* splicing is between 30% and 50% for a MMR gene,
      or
      iii) in case the allelic balance for a MMR gene is biallelic with a value of between 40% and 60%, wherein the protein sequence encoded by the FLT of said variant is predicted to be pathogenic or likely pathogenic (InSiGHT class 5 or 4), or
   (b2) the patient is determined not to suffer from Lynch syndrome due to a germline MMR gene defect, in case
      the allelic balance for the MMR gene is biallelic with a value of between 40% and 60%, wherein: i) the protein sequence encoded by the FLT of said variant is predicted to be benign or likely benign (InSiGHT class 1 or 2) or ii) said variant is a synonymous variant or intronic variant, and
      the degree of splicing is between 0% and 10% for said MMR exons, with the proviso of exon-specific splice variants of an MMR gene tolerated with following values for the degree of splicing: between 0% and 15% for skipping of *MLH1* exon 1q, *PMS2* exon 6p and exon 11, and between 0% and 25% for *MSH6* exon 4 (alternative splicing),
      or
      a DNA mismatch repair (MMR) variant is determined to be splice-neutral, in case
      the allelic balance for the respective MMR gene is biallelic with a value of between 40% and 60%,
      and
      the degree of splicing is between 0% and 10% for said MMR exons, with the proviso of exon-specific splice variants of an MMR tolerating following values for the degree of splicing between 0% and 15% for skipping of *MLH1* exon 1q, *PMS2* exon 6p and exon 11, and between 0% and 25% for *MSH6* exon 4 (alternative splicing).

In case the cells in the sample are incubated and/or cultured with puromycin in the culturing medium conditions, the nonsense-mediated mRNA decay (NMD) pathway is inactive in the cells.

Accordingly, in one preferred embodiment of the method of the invention, in steps (ai) and (aii), the nonsense-mediated mRNA decay (NMD) pathway is inactive in the cells in said sample. Therefore, in this embodiment, the cells of the sample are maintained and/or cultured in the presence of puromycin, such as in the presence of puromycin for at least 1, 2, 3, 4 or 5 hours, such as up to 10 or 20 hours.

In a more preferred embodiment, step (b) comprises, for the alternative of an inactive NMD pathway:
(b) diagnosing Lynch Syndrome or determining the pathogenicity of DNA mismatch repair (MMR) gene variants, or determining the mechanism of pathogenic DNA mismatch repair (MMR) gene defects or Lynch Syndrome, in the patient, wherein:
   (b1) Lynch Syndrome is diagnosed or a DNA mismatch repair (MMR) gene variant is determined to be pathogenic or likely pathogenic, or not to be splice-neutral,
      i) in case the allelic balance for a MMR gene is monoallelic with a value of between 0% and 10%, or
      ii) in case the allelic balance for a MMR gene is biallelic with a value of between 40% and 60%, and
         the degree of splicing is between 30% and 50% for the exons of said MMR gene, or
      iii) in case the allelic balance for a MMR gene exhibits allelic reduction with a value of >10% and <40%,
         and the degree of splicing is >10% and <30% for the exons of said MMR gene, or
      iv) in case the allelic balance for a MMR gene is biallelic with a value of between 40% and 60%, wherein the protein sequence encoded by the FLT of said variant is predicted to be pathogenic or likely pathogenic (InSiGHT class 5 or 4),
   (b2) the patient is determined not to suffer from Lynch syndrome due to a germline MMR gene defect, in case
      the allelic balance for a MMR gene is biallelic with a value of between 40% and 60%, wherein: i) the protein sequence encoded by the FLT of said variant is predicted to be benign or likely benign (InSiGHT class 1 or 2) or ii) said variant is a synonymous variant or intronic variant, and
      the degree of splicing is between 0% and 10% for the MMR exons, with the proviso of exon-specific splice variants of an MMR gene tolerating following values for the degree of splicing: between 0% and 15% for *MLH1* exon 1q, *PMS2* exon 6p, and *PMS2* exon 11, and between 0% and 25% for *MSH6* exon 4,
      or
      a DNA mismatch repair (MMR) variant is determined to be splice-neutral, in case
      the allelic balance for the respective MMR gene is biallelic with a value of between 40% and 60%,
      and
      the degree of splicing is between 0% and 10% for said MMR exons,
      with the proviso of exon-specific splice variants of an MMR tolerating following values for the degree of splicing between 0% and 15% for skipping of *MLH1* exon 1q, *PMS2* exon 6p and exon 11, and between 0% and 25% for *MSH6* exon 4 (alternative splicing).

In a yet further preferred embodiment, Lynch Syndrome is diagnosed or a DNA mismatch repair (MMR) variant is determined to be pathogenic or likely pathogenic, or not to be splice-neutral, in alternative (b1)(ii) or (iii), in case said splicing is determined to be specific for one allele, thus exhibiting allele-specificity.

It was further found that the accuracy of diagnoses can be further enhanced if the methods are performed both with cells maintained and/or cultured in the presence of puromycin, and cells maintained and/or cultured in the absence of puromycin.

For example, a sample may be obtained from a patient, and, after optionally culturing the cells, a part of the cells may be further cultured in the presence of puromycin, and another part of the cells may be further cultured in the absence of puromycin, as demonstrated in the examples in parallel approaches. Alternatively, two different samples may obtained from the same patient and used for culturing the cells of the first sample in the presence of puromycin, and culturing the cells of the second sample in the absence of puromycin.

Therefore, in a more preferred embodiment of the present invention:
(B1) Lynch Syndrome is diagnosed or a DNA mismatch repair (MMR) variant is determined to be pathogenic in case the criteria in (b1) of the embodiment with active NMD pathway are fulfilled and the criteria in (b1) of the embodiment with inactive NMD pathway are fulfilled, or
(b2) the patient is determined not to suffer from Lynch syndrome due to a germline MMR-defect or a DNA mismatch repair (MMR) variant is determined to be splice-neutral, in case the criteria in (b2) of the embodiment with active NMD pathway are fulfilled and the criteria in (b2) of the embodiment with inactive NMD pathway are fulfilled.

In a more preferred embodiment, diagnosing Lynch Syndrome or determining the pathogenicity of DNA mismatch repair (MMR) variants, or determining the mechanism of pathogenic DNA mismatch repair (MMR) gene defects or determining the mechanism of Lynch Syndrome comprises identifying a splice site defect (SSD) in at least one full-length transcript (FLT) of a MMR gene, in particular wherein a MMR gene variant is of InSiGHT class 3 (uncertain significance), class 4 (likely pathogenic) or class 5 (pathogenic) with causal connection to the SSD or the SSD is caused by an unknown molecular mechanism/variant.

A SSD caused by an unknown molecular mechanism/variant encompasses, for example, large genomic inversions, deep intronic variants, variants resulting in a regulatory defect or inducing promoter methylation, or resulting in mRNA instability.

In particular, it was demonstrated in the examples that previously unknown splice site defects (SSD) could be identified.

SSDs include, for example, the complete skipping of an exon, a partial exon skipping, such at the 5'end or the 3'end of the exon, the inclusion of an intron or parts of an intron, or a pseudo-exon inclusion. Moreover, it was demonstrated in the examples that, in several cases, the pathogenicity of variants previously classified as "Variant of Uncertain Significance (VUS)" could be determined.

Therefore, in a more preferred embodiment, diagnosing Lynch Syndrome or determining the pathogenicity of DNA mismatch repair (MMR) gene variants, or determining the mechanism of pathogenic DNA mismatch repair (MMR) gene defects or determining the mechanism of Lynch Syndrome comprises determining the pathogenicity of a DNA mismatch repair (MMR) gene variant which is a Variant of Uncertain Significance (VUS).

Moreover, it was demonstrated in the examples that the effect of splicing of a DNA mismatch repair (MMR) variant of InSiGHT class 3, 4 or 5 on the full-length transcript (FLT) could be determined. For example, determining the protein sequence encoded by the aberrantly spliced isoforms allows for determining their pathogenicity when the protein sequence encoded by the isoform is predicted to be pathogenic or likely pathogenic (InSiGHT class 5 or 4).

Therefore, in a more preferred embodiment, diagnosing Lynch Syndrome or determining the pathogenicity of DNA mismatch repair (MMR) gene variants, or determining the mechanism of pathogenic DNA mismatch repair (MMR) gene defects or determining the mechanism of Lynch Syndrome comprises determining the effect of splicing of a DNA mismatch repair (MMR) variant of InSiGHT class 3, 4 or 5 on the full-length transcript (FLT).

Moreover, it was demonstrated in the examples that the effect of a germline MMR gene defect by detecting an allelic loss in at least one DNA mismatch repair (MMR) gene could be determined.
With this method, also the deleterious effect of unknown genomic alterations in the MMR genes can be identified either by the detection of a SSD or an abnormal sequence in the FLT, such as an exon deletion caused by an inversion within one gene, or by demonstration of an allelic loss, which can be due to a lost transcript integrity of the FLT, e.g. due to large genomic inversions disrupting the FLT sequence and possibly creating new fusion transcripts, or to a regulatory defect in transcription, e.g. conferred by promoter variants or promoter hypermethylation.

Therefore, in a more preferred embodiment, diagnosing Lynch Syndrome or determining the pathogenicity of DNA mismatch repair (MMR) gene variants, or determining the mechanism of pathogenic DNA mismatch repair (MMR) gene defects or determining the mechanism of Lynch Syndrome comprises determining the effect of a germline MMR gene defect by detecting an allelic loss or aberrant splicing in at least one DNA mismatch repair (MMR) gene, in particular wherein the MMR gene variant is selected from a variant of InSiGHT class 3 (uncertain significance), class 4 (likely pathogenic) or class 5 (pathogenic) or caused by an unknown molecular mechanism/variant.

Further, it was surprisingly possible in the examples to determine a patient not to suffer from Lynch syndrome due to a germline MMR gene defect, even though the tumor indicates a MMR-deficiency by an immunohistochemical loss of at least one MMR protein.

Therefore, in one preferred embodiment of the methods of the invention, the patient is determined in step (b2) of a method of the present invention not to suffer from Lynch syndrome due to a germline MMR-defect, even though the tumor indicates a MMR-deficiency by an immunohistochemical loss of at least one MMR protein.

In particular, an immunohistochemical loss of MLH1 is typically accompanied by a loss of PMS2 and may indicate a germline defect in *MLH1.* Further, an isolated immunohistochemical loss of PMS2 may indicate a germline defect in *PMS2.* Further, an immunohistochemical loss of MSH2 is usually accompanied by a loss of MSH6 and may indicate a germline defect in *MSH2.* Further, an isolated immunohistochemical loss of MSH6 may indicate a germline defect in *MSH6.*

In a preferred embodiment, in case a patient has an informative variant in the gene which is indicated to be deficient by immunohistochemical loss in the tumor, the patient is determined not to suffer from Lynch syndrome due to the suspected germline MMR gene defect or a DNA mismatch repair (MMR) gene variant is determined to be splice-neutral, in case the allelic balance for the respective MMR gene is biallelic with a value of between 40% and 60% allelic balance (class 1-2), and the degree of splicing is between 0% and 10% for the MMR exons, and exon-specific exceptions tolerating higher values: up to 15% is tolerated for skipping of *MLH1* exon 1q, or *PMS2* exon 6p and exon 11, and up to 25% for *MSH6* exon 4, representing alternative splicing.

In a preferred embodiment, in case a patient suspected of having LS has an informative variant for all four MMR genes, the patient is determined not to suffer from Lynch syndrome due to a germline MMR-defect or a DNA mismatch repair (MMR) variant is determined to be splice-neutral, in case the allelic balance for each MMR gene is biallelic with a value of between 40% and 60% allelic balance (class 1-2), and the degree of splicing is between 0% and 10% for each MMR exon, and exon-specific exceptions tolerating higher values: up to 15% is tolerated for skipping of *MLH1* exon 1q, or *PMS2* exon 6p and exon 11, and up to 25% for *MSH6* exon 4 (alternative splicing).

In a yet further embodiment, the present invention relates to a set of primer pairs suitable for amplifying full-length transcripts (FLTs) or cDNA molecules generated thereof of the DNA mismatch repair (MMR) genes *MLH1, MSH2, MSH6* and *PMS2,*
wherein at least one primer pair is suitable for amplifying full-length transcripts (FLTs) or cDNA molecules generated thereof of *MLH1,* at least one primer pair is suitable for amplifying full-length transcripts (FLTs) or cDNA molecules generated thereof of *MSH2,* at least one primer pair is suitable for amplifying full-length transcripts (FLTs) or cDNA molecules generated thereof of *MSH6* and at least one primer pair is suitable for amplifying full-length transcripts (FLTs) or cDNA molecules generated thereof of *PMS2.*

In a yet further embodiment, the invention relates to the use of the set of primer pairs of the invention for diagnosing Lynch Syndrome, or for determining the pathogenicity of DNA mismatch repair (MMR) variants, or for determining the mechanism of pathogenic DNA mismatch repair (MMR) gene defects or Lynch Syndrome, in a patient suffering from Lynch Syndrome, suspected to suffer from Lynch Syndrome or having a risk to develop Lynch Syndrome, and/or for performing a method of the present invention described herein.

In a preferred embodiment, a primer pair suitable for amplifying full-length transcripts (FLTs) or cDNA molecules generated thereof of *MLH1* comprises a primer which comprises a sequence comprising at least 18, 19, 20, 21, 22, 23 or 24 contiguous nucleotides which is identical to the sequence within the 5' untranslated region (UTR) of the first exon of *MLH1,* or a sequence complementary thereto, and at least one reverse primer which comprises a sequence comprising at least 18, 19, 20, 21, 22, 23 or 24 contiguous nucleotides which is identical to the reverse complement of a sequence within the 3' UTR of the last exon of the longest transcript of *MLH1* or a sequence complementary thereto.

In a preferred embodiment, a primer pair suitable for amplifying full-length transcripts (FLTs) or cDNA molecules generated thereof of *MLH2* comprises a primer which comprises a sequence comprising at least 18, 19, 20, 21, 22, 23 or 24 contiguous nucleotides which is identical to the sequence within the 5' untranslated region (UTR) of the first exon of *MLH2,* or a sequence complementary thereto, and at least one reverse primer which comprises a sequence comprising at least 18, 19, 20, 21, 22, 23 or 24 contiguous nucleotides which is identical to the reverse complement of a sequence within the 3' UTR of the last exon of the longest transcript of *MLH2* or a sequence complementary thereto.

In a preferred embodiment, a primer pair suitable for amplifying full-length transcripts (FLTs) or cDNA molecules generated thereof of *MSH6* comprises a primer which comprises a sequence comprising at least 18, 19, 20, 21, 22, 23 or 24 contiguous nucleotides which is identical to the sequence within the 5' untranslated region (UTR) of the first exon of *MSH6,* or a sequence complementary thereto, and at least one reverse primer which comprises a sequence comprising at least 18, 19, 20, 21, 22, 23 or 24 contiguous nucleotides which is identical to the reverse complement of a sequence within the 3' UTR of the last exon of the longest transcript of *MSH6,* or a sequence complementary thereto.

In a preferred embodiment, a primer pair suitable for amplifying full-length transcripts (FLTs) or cDNA molecules generated thereof of *PMS2* comprises a primer which comprises a sequence comprising at least 18, 19, 20, 21, 22, 23 or 24 contiguous nucleotides which is identical to the sequence within the 5' untranslated region (UTR) of the first exon of *PMS2,* or a sequence complementary thereto, and at least one reverse primer which comprises a sequence comprising at least 18, 19, 20, 21, 22, 23 or 24 contiguous nucleotides which is identical to the reverse complement of a sequence within the 3' UTR of the last exon of the longest transcript of *PMS2* or a sequence complementary thereto.

In the examples, the *MLH1* transcript of 2.452 bp is amplified with a forward primer (located at c.-148_-129) TGTCCAATCAATAGCTGCCG (SEQ ID No: 2) and a reverse primer (located at c.*15_*33) AGAACACATCCCACAGTGC (SEQ ID No: 3). The *MSH2* transcript of 2.965 bp is amplified with a forward primer (located at c.-41_-22) GTCGCGCATTTTCTTCAACC (SEQ ID No: 4) and a reverse primer (located at c.*103_*121) AAGTTGATAGCCCATGGGC (SEQ ID No: 5). The *MSH6* transcript of 4.163 bp is amplified with a forward primer (located at c.-37_-19) CGTCCGACAGAACGGTTGG (SEQ ID No: 6) and a reverse primer (located at c.*24_*43) CCACCTTTGTCAGAAGTCAAC (SEQ ID No: 7). The *PMS2* transcript of 2.775 bp is amplified with a forward primer (located at c.-9_13) GTTGCATCCATGGAGCGAGCTG (SEQ ID No: 8) and a reverse primer (located at c.*157_*177) TTGCAAGCAATGCTCCATCTG (SEQ ID No: 9).

Accordingly, in a preferred embodiment, a set a primer pairs suitable for amplifying full-length transcripts (FLTs) or cDNA molecules generated thereof comprises a primer pair pursuant to SEQ ID Nos: 2 and 3, a primer pair pursuant to SEQ ID Nos: 4 and 5, a primer pair pursuant to SEQ ID Nos: 6 and 7 and a primer pair pursuant to SEQ ID Nos: 8 and 9.

The preferred embodiments for the methods of the invention also apply to the set of primer pairs and uses of the invention. The preferred embodiments for the set of primer pairs of the invention also apply to the methods and uses of the invention. The above methods and uses of the invention are preferably *in vitro* methods and uses.

In a further embodiment, the present invention relates to a method for diagnosing, treating, preventing or monitoring Lynch Syndrome, comprising performing a method of the invention, wherein in case Lynch Syndrome is diagnosed or a DNA mismatch repair (MMR) variant is determined to be pathogenic or likely pathogenic, or not to be splice-neutral, the patient is treated with a treatment selected from surgery to remove the tumor from the affected organ(s), in particular the colon, a therapeutically effective amount of a 5-fluorouracil-based adjuvant therapy, a therapeutically effective amount of an antibody directed a checkpoint inhibitor, in particular an anti-PD-1 antibody, and high dose acetylsalicylic acid treatment and/or wherein the patient is undergoing regular monitoring of the colon.

### Abbreviations

AF - allelic frequency
CRC - colorectal cancer
Δ/del - deletion
FLT - full-length transcript
IHC immunohistochemistry
if - in-frame
LR-RT PCR - Long-Range Reverse-Transcriptase Polymerase Chain Reaction
LS - Lynch Syndrome
MMR - DNA mismatch repair
MSI-H - high grade of microsatellite instability
cDNA-P - cDNA without puromycin incubation
NMD - nonsense-mediated mRNA decay
oof - out-of-frame
cDNA +P - cDNA with puromycin incubation
PTC - premature termination codon
SSD - splice site defect
VUS - variant of uncertain significance, class 3 by InSiGHT classification rules

### Figures

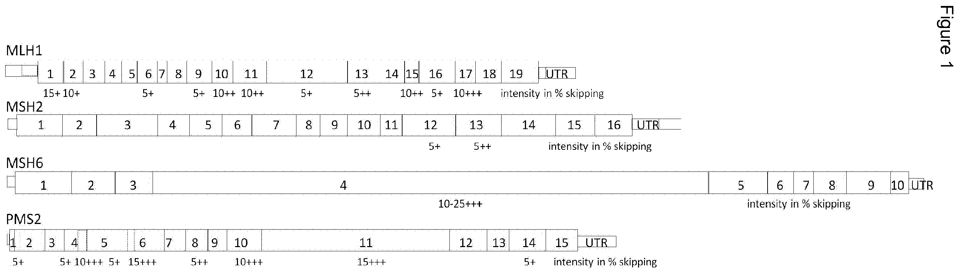

**Figure 1****:** Alternative splicing of a complete exon or partial exon is depicted for the four MMR transcripts with exon numbers, which we detected by LR-PCR in ten MMR-proficient controls with the respective intensity of exon skipping given in%, and frequency + indicates presence in one or two samples, ++ presence in 3-6 samples, and +++ for presence in 7-10 control samples in cDNA+P.

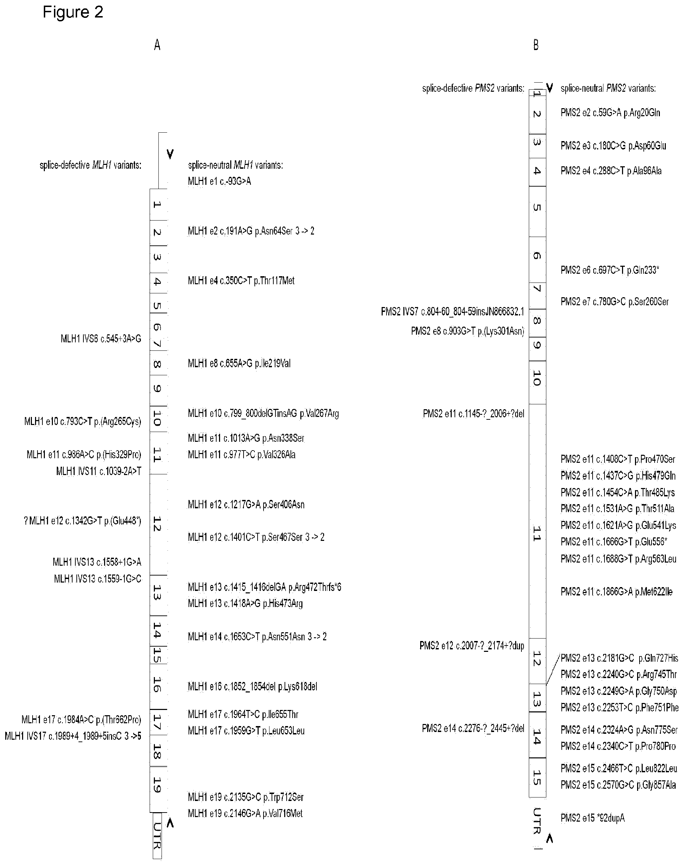

**Figure 2 A-D****:** Variants in the four MMR genes *MLH1, PMS2, MSH2,* and *MSH6* with variants designated as splice-defective on left side of the complete transcript with exons numbered, and splice-neutral variants on the right side by our LR-RT-PCR analyses. Arrows indicate primers used for LR-PCR.

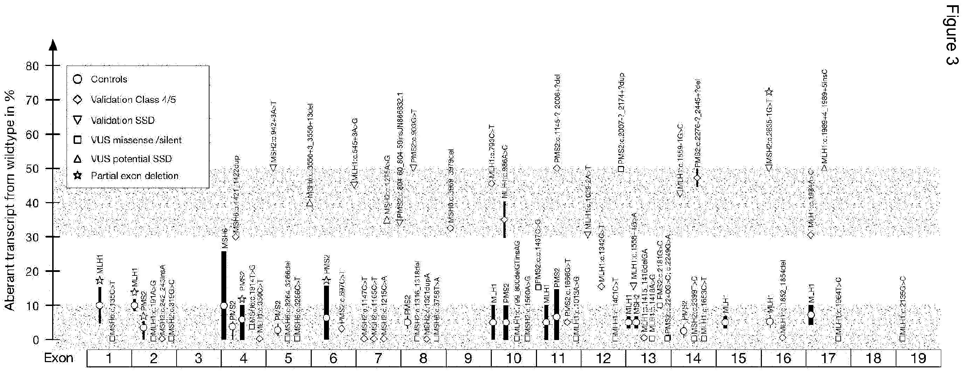

**Figure 3****:** Schematic allocation of variants in the four MMR transcripts sorted by exon numbers with splicing intensities in % in cDNA+P we detected with our method. Exon skipping in controls was regarded as alternative splicing, usually ranged 0-10% with a few exon-specific exceptions. In samples with a designated splice site defect aberrant splicing was expected, and usually ranged 30-50%. We therefore defined cut off values for alternative splicing up to 10% in the lower grey zone (allowing exon-specific exceptions with higher values), and for aberrant splicing at least from 30 to 50% in the upper grey zone. Then, we investigated splicing of the variant-bearing exon in samples with a PTC, pathogenic missense variant, or VUS including potential SSD, predicted missense or synonymous variant. * indicates a partial exon skipping.

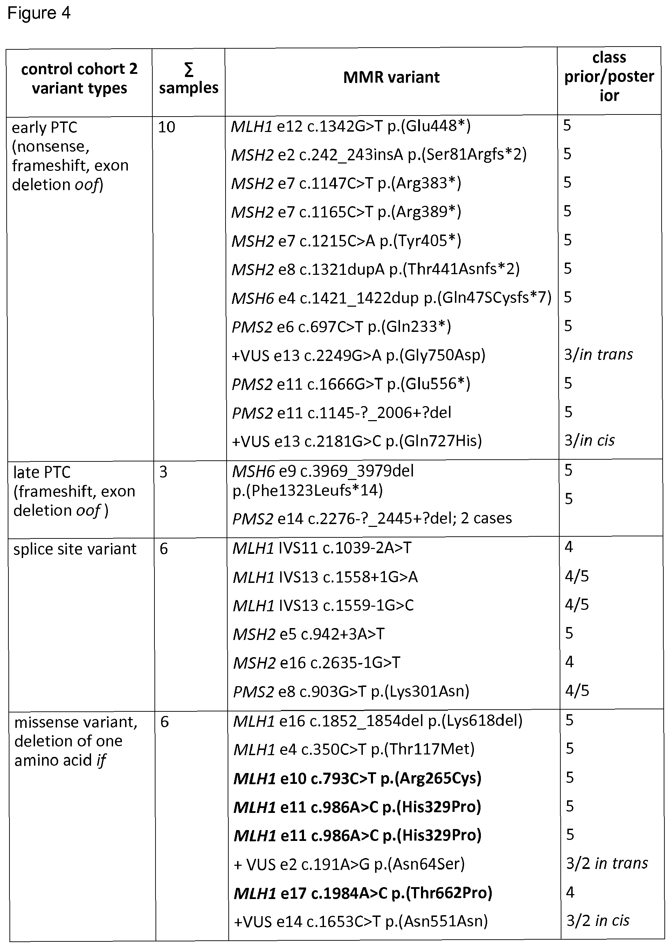

**Figure 4****:** shows Table 1: Control cohort 2 was selected upon presence of a variant in the MMR gene exon (e) or intervening sequence (IVS) with a classification as 4 (likely pathogenic) or 5 (pathogenic) according to the LOVD database or InSiGHT classification rules. Four cases with a pathogenic variant additionally harbour a class 3 VUS indicated as (+), for which the allelic position *in cis* or *in trans* was determined by cDNA analyses. Prior/posterior indicates the classification according InSiGHT before and after cDNA analyses. PTC: premature termination codon, oof: out-of-frame, *if*: in-frame. Variants in bold represent supposed missense variants which turned out to be splice defects.

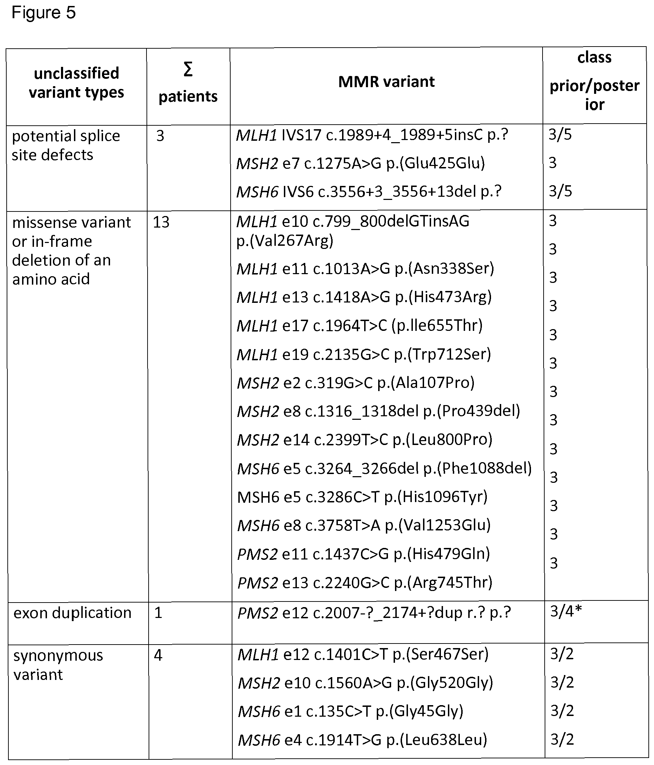

**Figure 5****:** shows Table 2: The 21 MMR gene variants of uncertain significance (class 3) with location given in consecutively numbered exon (e) or intervening sequence (IVS) were selected for the patient cohort "class 3 variant group" to investigate their effect on splicing and allelic expression in cDNA samples. Prior/posterior indicates the classification according to InSiGHT before and after cDNA analyses, * not according to InSiGHT guidelines.

### Examples

### Materials and methods

### Study cohort

All patients provided their informed consent for cancer genetics research compliant with ethical standards. Patients were selected on the following criteria: Bethesda positive, with either deficiency for at least one MMR-protein in the immunohistochemical staining (IHC) of the tumor or fulfilling the Amsterdam criteria with no tumor available for IHC or microsatellite instability (MSI) testing (Vasen, et al., 1999). In case of an MLH1 protein loss, the promoter methylation of *MLH1* was investigated by methylation-sensitive multiplex ligation-dependent probe amplification MS-MLPA (MRC Holland) in blood and tumor DNA (Morak, et al., 2018; Morak, et al., 2008).

*Control cohort 1:* The control cohort 1 included samples from patients with MMR-proficient tumors and was used to evaluate the normal physiological splicing for the MMR transcripts. For each MMR transcript, ten samples were analysed, and all had benign informative sequence variants in *MLH1, MSH6* or *PMS2.* For *MSH2,* where coding variants are extremely rare, only three of the ten samples had an informative variant.

*Control cohort 2:* The control cohort 2 comprised 25 samples from patients with a likely pathogenic (class 4) or pathogenic (class 5) sequence variant (shown in table 1). To test a successful NMD-inhibition we analysed ten samples with predicted early PTC variants, and three samples with predicted late PTC. For defining the cut off value for aberrant splicing we analysed six cDNA samples harbouring designated splice site variants. Six samples with five missense variants and one deletion of one amino acid (class 4-5) were investigated to discriminate pathomechanisms on RNA and protein level.

*Patient cohort 1:* The patient cohort 1 was composed of 21 patients with suspected LS and a class 3 variant in one of the MMR genes (table 2) including potential splice site variants, missense variants or deletions of one amino acid, synonymous variants, and an exon duplication ("class 3 variant group") to test the method the way it is supposed to work on VUS.

*Patient cohort 2:* The 26 patients of cohort 2 fulfilled at least the revised Bethesda criteria and showed a loss of MMR protein staining in the tumor IHC without any potentially causative germline sequence alteration (class 3-5) detectable but harbouring a heterozygous benign variant (class 1) in the target MMR transcript ("with unsolved MMR-deficiency"). In detail, an IHC loss was reported for MLH1/PMS2 in 16 patients, for PMS2 in five patients, for MSH2/MSH6 in two patients, and for MSH6 in three patients.

### cDNA preparation

The lymphocytes were separated from LeucoSept tubes (greiner bio-one), cultured for 72-96 h at 37°C, and incubated for 5 h at 37°C in parallel approaches without and with puromycin according to (Andreutti-Zaugg, et al., 1997; Vreeswijk and van der Klift, 2012), denominating in cDNA-P and cDNA+P. Total RNA was extracted from cultured lymphocytes following the instructions of the QIAamp RNA blood mini kit (QIAGEN), which includes a DNase digestion. For many samples, RNA was also isolated using the PAXgene blood RNA extraction kit (PreAnalytics) (Vreeswijk and van der Klift, 2012).

For cDNA synthesis, 1 µg of total RNA was subjected to the iScript Select cDNA synthesis kit (Bio-Rad) and Oligo-(dT)₁₈ primer (SEQ ID No: 1) following the manufacturer's instructions, however, we altered the incubation temperature to 48°C and extended the incubation time to 75-90 min. A test PCR for feasibility of cDNA amplification was carried out by a short PCR with primers in *MLH1* exon 7 forward and exon 8 reverse, which in parallel ensured absence of genomic contamination if only the cDNA fragment of 132 bp was present in the agarose gel lacking the corresponding genomic fragment with 280 bp (Morak, et al., 2011).

### cDNA amplification and sequence analysis

From 1-2 µl cDNA a Long-Range RT-PCR (LR-PCR) was carried out for the transcript of *MLH1, MSH2, MSH6,* or *PMS2* with the forward primer located in the 5' untranslated region (UTR) of the first exon, and the reverse primers in the 3'UTR of the last exon of the longest transcript. To enable allelic discrimination for as many samples as possible, we included the *MLH1* promoter variant c.-93G>A with an allelic frequency (AF) of 0.441 in Europeans, which made it necessary to establish the LR-PCR for *MLH1* for the isoform with the longest 5'UTR, as described in Genbank. Further benign variants with considerable AF located in the coding regions of *MLH1, MSH6* and *PMS2* were used to investigate the allelic balance of expression. For annotation we used the RefSeq FLT NM_000249.3, NG_007109.2 for *MLH1,* NM_000251.2, NG_007110.2 for *MSH2,* NM_000179.2, NG_007111.1 for *MSH6,* and NM_000535.5, NP_000526.2 for *PMS2.*

The *MLH1* transcript of 2.452 bp is amplified with a forward primer (located at c.-148_-129) TGTCCAATCAATAGCTGCCG (SEQ ID No: 2) and a reverse primer (located at c.*15_*33) AGAACACATCCCACAGTGC (SEQ ID No: 3) using IProof High-Fidelity DNA Polymerase (BioRad). Annealing temperature was 61°C for 30 sec, and extension time was 1 min 20 sec in 39 cycles.

The *MSH2* transcript of 2.965 bp is amplified with a forward primer (located at c.-41_-22) GTCGCGCATTTTCTTCAACC (SEQ ID No: 4) and a reverse primer (located at c.*103_*121) AAGTTGATAGCCCATGGGC (SEQ ID No: 5) using IProof High-Fidelity DNA Polymerase (BioRad). Annealing temperature was 59°C for 30 sec, and extension time was 1 min 30 sec in 39 cycles.

The *MSH6* transcript of 4.163 bp is amplified with a forward primer (located at c.-37_-19) CGTCCGACAGAACGGTTGG (SEQ ID No: 6) and a reverse primer (located at c.*24_*43) CCACCTTTGTCAGAAGTCAAC (SEQ ID No: 7) using PrimeSTAR GXL DNA Polymerase (TAKARA Clontech). Annealing temperature was 60°C for 15 sec, and extension time was 4 min 30 sec in 30 cycles.

The *PMS2* transcript of 2.775 bp is amplified with a forward primer (located at c.-9_13) GTTGCATCCATGGAGCGAGCTG (SEQ ID No: 8) and a reverse primer (located at c.*157_*177) TTGCAAGCAATGCTCCATCTG (SEQ ID No: 9) using TaKaRa LA Taq DNA Polymerase (TAKARA Clontech) in a 2-step PCR program with 68°C for 2 min 30 sec in 30 cycles.

In particular samples, additional short PCR amplifications with selected primers followed by sequencing were performed to investigate allele-specificity of a SSD or the cis/trans status of variants.

The PCR products were visualised on a 1% agarose electrophoresis gel to check for successful FLT amplification and to evaluate the presence and quantity of additional isoforms. The LR-PCR amplification was successful for 104 of 106 lymphocyte culture cDNAs-P/+P each, and for 26 out of 73 cDNAs from PAXgene, indicating an insufficient quantity or quality of cDNA. The total PCR products were treated with Exo-SAP kit (USB) and directly subjected to Sanger sequencing with several internal primers to generate overlapping reads of 600-900 bp length in one direction by using Big Dye v1.1 (Applied Biosystems). We chose direct sequencing as for standard FLT analysis to avoid the loss of low-abundance fragments from gel-extraction, as previously described likewise (Sumitsuji, et al., 2003). Sequences were run on ABI PRISM 3100 Avant.

Visual inspection of the FLT sequences by eye, especially at known variants and exon-exon-junctions was performed to assess the allelic balance and the splicing pattern. In sequences with two or three lines we defined the additional sequences divergent from the wildtype, assigned their origin, and measured their peak heights using Sequence Scanner v2.0 (Applied Biosystems). The peak intensities were calculated in % as described below, rounded to intervals of 5, and were reported starting from 5%. First of all, known heterozygous variants in the transcript in cDNA-P were checked to detect allelic losses or ensure biallelic expression. The balance of the variant in the genomic sequence was tested for a 50% distribution, or otherwise adapted. In cDNA, the larger peak in a heterozygous variant was defined as one allele representing 50%, and the intensity of the lower peak was calculated accordingly. In a second step, the splicing patterns were assessed in cDNA-P and compared to cDNA+P. Isoforms become visible in addition to the FLT by presence of an additional sequence starting typically at exon boundaries (Vreeswijk and van der Klift, 2012). In cDNA with proven biallelic expression we calculated the intensities of FLT and alternative spliced isoforms so that they together represent 100%. For an automated back-up sequence analysis we used the Mutation Surveyor 3.1 (SoftGenetics) software.

Nomenclature is complying with HGVS standard recommendations (http://varnomen.hgvs.org/recommendations) (den Dunnen and Antonarakis, 2000) and refers to the genomic position in hg19. For SSDs we used the nomenclature Δ for complete skipping of an exon by giving its number, a partial exon skipping is indicated by p for the 5'end and q for the 3'end of the exon, and an intron or pseudo-exon inclusion is indicated as an insertion according to (Colombo, et al., 2014). We used the software Alamut Visual Version 2.10 (Interactive Biosoftware, France, http://www.interactive-biosoftware.com) to predict SSDs with five integrated algorithms (SpliceSiteFinder-Like, MaxEntScan, NNSPLICE, GeneSplicer and Human Splice Finder) in addition to the algorithms considered to predict the effect on protein function (Align GVGD, SIFT, Mutation Taster, MAPP, PolyPhen) as well as evolutionary nucleotide and amino acid conservation and AF in different populations obtained from the GnomAD browser and/or 1000genomes (Chan, et al., 2007). All variants and cDNA results were submitted to the InSiGHT MMR gene variant database LOVD3 (http://www.insight-database.org). In applying the 5-tiered InSiGHT guidelines (Thompson, et al., 2014) we categorised variants as class 5 (pathogenic), class 4 (likely pathogenic), class 3 (variant of uncertain significance, VUS), class 2 (likely not pathogenic), or class 1 (not pathogenic).

### Results

### cDNA analysis in MMR-proficient samples (Control cohort 1)

To define biallelic expression and the alternative splicing pattern of the each MMR transcripts, we investigated cDNA-P and cDNA+P of the MMR-proficient control cohort 1. The benign informative variants in ten control samples for *MLH1, MSH6* and *PMS2,* and three control samples for *MSH2* all showed a normal expression of both alleles with 40-60% of intensity. Alternative splicing of an exon was denominated as recurrent when found in at least two samples (depicted in Figure 1), and their consequence was documented as *in-frame* (*if*) or *out-of-frame* (*oof*) transcripts.

In detail, we detected several alternative isoforms for *MLH1* in cDNA-P with intensities of 5-10% (9 *oof*, 12 *oof,* 15 *oof*, 16 *if,* and most frequently 17 *if*) and in cDNA+P ranging 5-10% (2p r.117_121del oof, 6 *oof*, 9 *oof*, 12 *oof*, recurrent skipping of 10 *oof,* 11 *oof,* 15 *oof*, 16 *if,* 17 *if*)*.* In one cDNA+P sample exon 1q r.-62_116del p.? was skipped with 15% intensity. For *MSH2* we found only one sample in cDNA-P skipping exon 12 *if* with 5% intensity, and in cDNA+P we observed recurrent skipping of exon 13 *oof* with 5% intensity. *MSH6* showed a skipping of its huge exon 4 *oof* in most cDNAs-P with intensities of 5-10% and in all cDNAs+P usually ranging 10-15%, and in some cases to up to 20-25%. In *PMS2,* we detected diverse isoforms in cDNA-P with 5-10% (2p r.24_28del *oof,* 4 *oof,* 11 *oof,* recurrent skipping of 10 *if*), whereas in cDNA+P we observed even more isoforms with 5-10% (8 *oof,* 14 *oof,* recurrent skipping of 2p *oof,* 4 *oof,* 4q r.301_353 *oof,* 6p r.538_586del *oof,* 10 *if,* 11 *oof*). In one cDNA+P the partial skipping of exon 6p *oof* was 15%, and in another cDNA+P exon 11 showed 15% skipping.

Biallelic expression was defined with 50±10% allelic balance in heterozygous variants. Alternative splicing was generally lower in cDNA-P than in cDNA+P, and ranged in cDNA-P from 0-10% due to active NMD degrading isoforms with an early PTC. In cDNA+P alternative splicing was visible in a higher extent usually between 5-10% intensity, whereas some isoforms exceeded this value with presence up to 15% in *MLH1* or *PMS2,* and *MSH6* exon 4 up to 25%.

### cDNA analysis of samples with pathogenic variants (Control cohort 2)

The control cohort 2 included cDNA samples harbouring different types of pathogenic sequence variants (Table 1) to test the system for appropriate NMD and NMD inhibition, and to define cut off values for aberrant splicing by investigating variants generating a SSD. Furthermore, we investigated missense variants classified as pathogenic on protein level, to test whether their pathogenicity might actually be based on aberrant splicing.

### Nonsense mediated decay in class 4-5 early and late PTC variants

Successful NMD inhibition was tested by ten variants expected to cause NMD due to an early PTC. For each of the four major MMR transcripts at least one early PTC was investigated (1x *MLH1,* 5x *MSH2,* 1x *MSH6,* 3x *PMS2,* details see table 1). The cDNA-P sequences showed an almost monoallelic expression of the wildtype allele, the respective allele harbouring the early PTC was absent or strongly reduced with 0-10% intensity in all samples due to active NMD. In cDNA+P the early PTC allele was restored, showing intensities of 15%-40% for eight of the ten variants, as demonstrated for all four transcripts *MLH1, MSH2, MSH6,* and *PMS2.* Of those, one variant *MLH1* c.1342G>T p.(Glu448*) in exon 12 showed an enhanced alternative splicing of exon 12 in up to 15% and additional isoforms (5-10% skipping of exon 9-10 *if,* 9-12 *if,* 9-13 *oof*, 12 *oof*, 13-15 *oof*). Two of the five early PTCs in *MSH2* were only visible with maximum 5 or 10% intensity in forward and reverse sequences even though NMD-inhibition was carried out, and no additional aberrant splicing was detected. For these two samples with *MSH2* variants in exon 2 (c.242_243insA p.(Ser81Argfs*2)) and exon 7 (c.1215C>A p.(Tyr405*)) additional short PCRs from cDNA+P spanning exon 1-4, or 4-12, 5-8, and 7-11 showed the same underrepresentation of the early PTC allele as in the LR-PCR, and therefore an allelic loss due to primer selection seems unlikely.

We tested NMD evasion on two variants predicted to generate a late PTC in three samples. The heterozygous variant *MSH6* c.3969_3979del generated two isoforms in cDNA-P and +P, the expected partial exon 9q deletion r.3969_3979del p.Phe1323Leufs*14 and skipping of the entire exon 9 r.3802_4001del p.Ala1268Glyfs*6, both resulting in a late PTC with intensities between 15-35%. In two unrelated samples with genomic deletions of *PMS2* exon 14 c.2276-?_2445+?del the finding of a 50% skipping of exon 14 r.2276_2445del p.Ala759Glyfs*8 in cDNA-P/+P confirmed the location of the deletion within the gene, and not in one of the pseudogenes, resulting in a late PTC in exon 15.

Our cDNA results reassure that transcripts with a late PTC are not subjected to NMD. Transcripts with an early PTC were underrepresented with 0-10% in cDNA-P due to NMD and defined as an allelic loss, but can be restored up to 40% intensity in cDNA+P by NMD-inhibition in most of the cases.

### Aberrant splicing in class 4-5 splice site variants

To define cut off values for aberrant splicing, we analysed cDNAs with six variants designated as class 4 or 5 SSD in LOVD3 (intronic splice acceptor: *MLH1* c.1039-2A>T; c.1559-1G>C; *MSH2* c.2635-1G>T; splice donor: *MLH1* c.1558+1G>A*; MSH2* c.942+3A>T; exonic splice donor: *PMS2* c.903G>T).

In cDNA-P we detected aberrant *if* isoforms with 20-50% intensity for three variants (*MLH1* c.1039-2A>T, *MSH2* c.942+3A>T, and *MSH2* c.2635-1G>T). The other three SSD variants (*MLH1* c.1558+1G>A, *MLH1* c.1559-1G>C, and *PMS2* c.903G>T) were found with only 0-10% intensity in cDNA-P due to active NMD. The allelic loss of coding variants in these three samples was used as indicator for a complete SSD of the variant allele into isoforms subjected to NMD.

In cDNA+P we observed a high percentage of aberrant splicing (30-50%) of the respective exon for five of the six SSD variants (details see figure 1), and for one variant *MLH1* c.1558+1G>A the detection of only 15% aberrant intron inclusion was explained by an allelic reduction of the pathogenic allele.

In detail, two splice site variants exerted a complete skipping of the exon affected by the variant in cDNA+P, which was visible as 50% aberrant isoforms: *MSH2* c.942+3A>T induced *if* skipping of exon 5 (r.793_942del p.Val265_Gln314del), and *PMS2* c.903G>T caused *oof* skipping of exon 8 (r.804_903del p.Tyr268*) and enhanced alternative *oof* splicing of exon 6p (r.538_586del p.Glu180Glnfs*5) up to 15%. The exonic variant *PMS2* c.903G>T was absent in cDNA-P/+P because it functions as a complete SSD abrogating inclusion of exon 8 on its allele.

Two variants created several isoforms in cDNA+P (in total 30-50%) lacking the affected exon and also neighbouring exons: *MLH1* c.1039-2A>T in intron 11 caused an *oof* deletion of exon 12 (r.1039_1409del p.Thr347Lysfs*8) in 20%, and an *if* deletion of exon 9-12 (r.678_1409del p.Glu277_Arg470del) in 10%; *MLH1* c.1559-1 G>C skipped exon 14 (r.1559_1667del p.Leu521Lysfs*34) *oof* in 20-35%, exon 14-15 (r.1559_1731 del p.Val520Glyfs*7) *oof* in 15-20%, and enhanced splicing of exon 10-11 (r.791_1038del p.Arg265Phefs*14) *oof* with intensities ranging 10-20%.

One variant caused a partial exon deletion detectable in 50% in cDNA-P/+P leading to a late PTC: *MSH2* c.2635-1G>T induced a deletion of the first four nucleotides of exon 16 (r.2635_2638del p.Gln879Valfs*12).

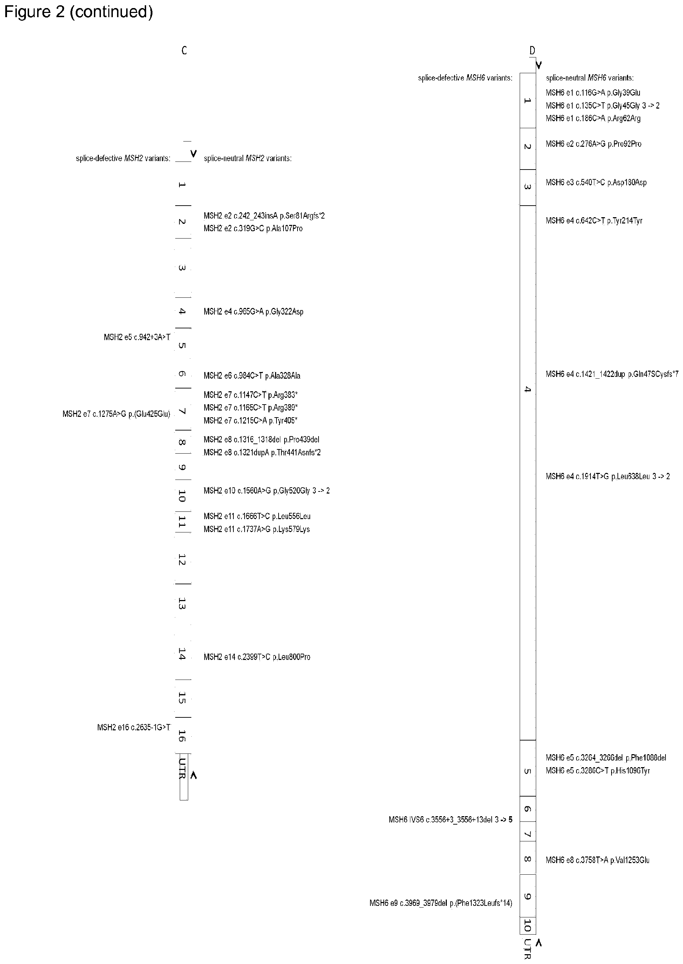

For one splice site variant a partial intron retention was observed with only 15% intensity in cDNA+P: *MLH1* c.1558+1G>A inserted intron 13 (r.1558_1559ins1558+1_1558+108 p.Val520Glyfs*3) and an enhanced alternative splicing of exon 9-10 with 15%. The informative variant c.655A>G in this sample showed only 10-15% intensity in cDNA+P for the G allele indicating an allelic reduction of the pathogenic allele and explaining the low percentage of aberrant splicing of 15%. In InSigHT database, another variant in the same location *MLH1* c.1558+1G>T was designated as class 5 SSD generating the same isoform with unknown intensity. Variants in the conserved splice sites can reliably be predicted as SSD, and aberrant splicing was visible with 30-50% intensity at least in cDNA+P with normal allelic balance, and this range was defined as aberrant splicing (Figure 2+3). The detection of an allelic underrepresentation of an informative variant (<40%) indicates an abnormality so that potential splice site aberrations cannot be assessed accordingly and need further investigation.

### class 4-5 missense variants or deletions of one amino acid

Six variants assumed to affect one amino acid and already classified as pathogenic were analysed in cDNA to test whether their pathogenicity is based on aberrant splicing even though not predicted, or on protein level. By checking the allelic balance in cDNA-P/+P the variant alleles of *MLH1* c.793C>T p.(Arg265Cys) and *MLH1* c.986A>C p.(His329Pro) showed an allelic loss with intensities of 0-5%, and *MLH1* c.1984A>C p.(Thr662Pro) was underrepresented with 20%. This can be explained by an extensive amount of aberrantly spliced isoforms detected especially in cDNA+P with intensities of 30-40% skipping also the exon affected due to the variant. In cDNA-P of these three cases aberrant *oof* transcripts were found with maximum 5% intensity due to NMD.

In detail, *MLH1* c.793C>T in exon 10 created several isoforms by skipping of exon 10 *oof*, 9-10 *if,* 9-11 *oof*, and 10-11 *oof* with intensities of 5-15% each in cDNA+P.

*MLH1* c.986A>C in exon 11 induced a skipping of exon 10-11 *oof* and 9-11 *oof* with intensities of 10-30%, but no isoform with isolated skipping of exon 11 was detected. In a second sample from another patient with *MLH1* c.986A>C and an additional VUS in *MLH1* exon 2 (c.191A>G p.(Asn64Ser)), the expression of this VUS was monoallelic (c.191G) in cDNA-P indicating an allelic loss of the other allele, and biallelic in cDNA+P showing absence of exon 10-11 *oof* with 30% intensity. The location of the variant *MLH1* c.191A>G p.(Asn64Ser) *in trans* to a pathogenic variant in absence of CMMRD features allows a reclassification of this VUS from class 3 to class 2.

For *MLH1* c.1984A>C in exon 17, an *if* skipping of the entire exon 17 (r.1897_1989del p.Glu633_Glu663del) was found with 10% intensity in cDNA-P, and 30% intensity in cDNA+P. As exon 17 is an alternatively spliced exon, we checked for an aberrant allele-specific splicing by analysing the VUS c.1653C>T p.(Asn551Asn) in *MLH1* exon 14 additionally present in the sample, which showed biallelical expression in cDNA-P/+P. By selecting for transcripts including exon 17, the c.1653T-allele was 30% reduced indicating an allelic status *in cis* of both variants and a partial, allele-specific SSD induced by the variant in exon 17. In addition, the synonymous VUS was shown to be splice-neutral, which allows a reclassification of c.1653C>T p.Asn551Asn from class 3 to class 2.

*MLH1* c.1852_1854del p.Lys618del and c.350C>T p.Thr117Met presented a balanced expression with 50% intensity in cDNA-P/+P in absence of aberrant splicing, and are designated as splice-neutral.

In conclusion, the three *MLH1* class 4-5 putative missense variants c.793C>T p.(Arg265Cys), c.986A>C p.(His329Pro), and c.1984A>C p.(Thr662Pro) are in fact SSDs (Figure 2+3) even though not located next to a splice site, and not predicted as SSD by bioinformatics. Interestingly, *in-silico* protein predictions indicated a disease-causing effect for *MLH1* c.1984A>C p.(Thr662Pro) in three of five algorithms and for *MLH1* c.793C>T p.(Arg265Cys) in all five.

### cDNA analysis for class 3 MMR variants (Patient cohort 1)

We applied our FLT analysis method to 21 class 3 MMR variants (Table 2) in patient cohort 1 to investigate their effect upon mRNA splicing, and depict the results for splicing of the variants in Figure 2+3.

### Potential SSD variants

Three class 3 variants *MLH1* c.1989+4_1989+5insC in intron 17, *MSH2* c.1275A>G p.(Glu425Glu) in exon 6, and *MSH6* c.3556+3_3556+13del in intron 6 potentially exert a SSD, as they are located close to the splice sites, show an evolutionary sequence conservation of 62-84%, and two to five algorithms predict a reduction or loss of the splice donor site.

In cDNA-P we detected aberrant *if* transcripts with 30-50% intensity for two variants, whereas the third variant presented an allelic loss with only 5% due to NMD of the aberrant *oof* transcript. In cDNA+P aberrant splicing was visible for all three variants with 35-50% intensity.

*MLH1* c.1989+4_1989+5insC generated an exon 17 *if* skipping (r.1897_1989del p.Glu633_Glu663del) with an intensity of 50% in cDNA-P/+P, and biallelic expression of the benign variant c.1217G>A p.Ser406Asn in exon 12. Allele-specific 100% skipping of exon 17 was shown by PCR from exon 11-17 selecting for transcripts including exon 17, where the benign variant allele c.1217A was monoallelically present.

*MSH2* c.1275A>G induced an *if* skipping of the rear part of exon 7 (r.1229_1276del p.lle411_Gly426del) with 30-35% intensity in cDNA-P/+P. In reverse sequencing of exon 7 the variant c.1275G appeared to be still present with 15% intensity indicating an allele-specific, but incomplete splicing defect of the SSD variant.

The cDNA-P sample of *MSH6* c.3556+3_3556+13del showed an allelic loss of the benign variant c.116A in exon 1 due to NMD. In cDNA+P a 50% *oof* skipping of exon 6 (r.3439_3556del p.Ala114Valfs*9) was detected, and the benign variant c.116G>A p.Gly39Glu was biallelically expressed with 50%.

To sum up, we found aberrant splicing with intensities of 30-50% for all three potential SSD VUS. Two variants were designated as complete SSDs, which allowed a re-classification to class 5 (pathogenic), whereas the third variant was a significant but partial SSD regarded as class 3.

### Missense variants or small in frame deletions

Based on a balanced biallelic expression (45-60%) and absence of aberrant splicing in cDNA-P/+P for all eleven missense variants and two variants deleting one amino acid (table 2), we designated these variants as splice-neutral. However, for re-classification of these variants the effects on protein level have to be clarified in a consented manner.

### Exon duplication

The FLT analysis of a sample with a genomic duplication in *PMS2* exon 12 of unknown localisation showed an *if* insertion r.2007_2174dup p.Lys670_Ala725dup with 50% intensity in cDNA-P/+P verifying the disruption of one allele. Even though InSiGHT rules would conclude class 3 for this *if* insertion, we regard it as class 4 because it occurs in the protein domain of functional importance for MLH1 interaction.

### Synonymous variants

Four synonymous variants showed a biallelic expression with intensities of 50-60%, and a normal splicing pattern with a maximum of 5% for skipping the exon harbouring the variant or any exon in cDNA-P/+P. Even for the variant located in *MSH6* exon 4, which is known for elevated levels of alternative splicing in controls, the exon 4 skipping was only 5%. We therefore designated these four synonymous variants *MLH1* c.1401C>T p.Ser467Ser, *MSH2* c.1560A>G p.Gly520Gly, *MSH6* c.135C>T p.Gly45Gly, c.1914T>G p.Leu638Leu as splice-neutral, which enables a re-classification from class 3 to class 2.

### cDNA analysis in unsolved patients with MMR-deficient tumors

We performed FLT analyses for 26 patients with molecularly unexplained MMR-deficiency in their tumor and clinical suspicion of LS, in which routine diagnostics did not detect a class 3-5 germline MMR variant. Their corresponding tumor IHC indicated a defect of MLH1 in 16 patients, of MSH2 in two, of MSH6 in three, and of PMS2 in five patients. By investigating the allelic balance of a benign, heterozygous variant (class 1) in the relevant FLT of *MLH1, MSH2, MSH6,* or *PMS2* we performed a screening for an allelic loss or aberrant splicing pattern.

An equal intensity of benign variant alleles ranging between 45-55% and a normal splicing pattern was found in cDNA-P/+P for 21 of the 26 patients. In one of those patients, our cDNA analysis for *PMS2* additionally detected a 50% heterozygosity in five benign variants in exon 13, 14, 15 and the 3'UTR, which were not found in germline analysis performed according to (Clendenning, et al., 2006) due to an allelic loss.

In cDNA-P of five patients, an allelic loss was detected with intensity of one allele below of 10%, four indicating a defect in *MLH1,* and one in *PMS2.* In cDNA+P of three of these patients the NMD-inhibition restored the missing allele with an allelic presence of 30-50% in benign variants. Furthermore, the explanatory pathogenic cDNA changes were visible in cDNA+P sequencing: an early PTC variant in *MLH1* exon 13 (r.1415_1416delGA p.Arg472Thrfs*6) with 50% intensity, an *oof* skipping of exon 6 in *MLH1* (r.454_545del p.Thr152Leufs*3) with 45% intensity, and a *PMS2* insertion of 71 nucleotides (r. 803_804insAATGTGCCATGTGAACCACCCCGTCTGAAAAGTGAGGAGCCCCT CTGCCCGGCAGC CGCCCCGTCTGGGAG p.Tyr268*) (SEQ ID No: 10) prior to exon 8 with 35% intensity. In cDNA+P of two patients the allelic loss of *MLH1* remained, and was confirmed in new, independent blood samples, but the reasons for this still need to be discovered.

### Genomic sequencing of selected patients with unexpected cDNA alterations

To search for the underlying mechanisms of the conspicuous cDNA results in five patients, genomic re-sequencing of respective exons and introns was performed. Two pathogenic germline variants were detected, which were initially overlooked in earlier routine diagnostics screening: the early PTC *MLH1* c.1415_1416delGA p.Arg472Thrfs*6, and the splice site variant *MLH1* c.545+3A>G explanatory for exon 6 skipping. A large genomic SVA-insertion of 2.2 kb in *PMS2* described previously (van der Klift, et al., 2012) was found as the underlying genetic cause for the insertion *oof* on cDNA level. For the other two patients with a remaining allelic loss of *MLH1* in cDNA+P and inconspicuous genomic re-sequencing a regulatory or structural defect has to be assumed. While beyond the scope of this project, further investigations will be necessary to determine the underlying mechanisms.

### Method standardisation

Despite refined bioinformatical algorithms, the *in-silico* prediction of a SSD is only fairly reliable even for variants within the splice sites. Therefore, experimental testing of variants for potential splicing aberrations is needed, even though being tedious. We comprehensively describe a robust protocol for the FLT analysis of the four major MMR genes *MLH1, MSH2, MSH6* and *PMS2* from patients' heparin samples in short-term lymphocyte culture with/without puromycin incubation prior to RNA isolation, cDNA synthesis, LR-PCR amplification of the FLT and direct Sanger sequencing. The amplification of FLT requires a good cDNA quality in length and abundance which allowed analysis of 98% of cDNAs from lymphocyte culture and 36% of cDNAs from PAXgene. Our protocol was validated by defining gene- and exon-specific cut off values for alternative and aberrant splicing, and by investigating the allelic balance in informative variants. The FLT analysis is universally applicable to examine the effect of variants upon splicing, which helps to classify VUS and generally should be conducted prior to functional protein assays. Furthermore, this method can be used to test the FLT integrity in unsolved patients by using an informative variant, and is able to detect an allelic loss as well as other defects.

To our knowledge, this is the first report of a FLT PCR protocol at least for *MSH6* and *PMS2.* Previously, FLT amplification and analysis was published for *MLH1* and *MSH2* (Etzler, et al., 2008) (Sumitsuji, et al., 2003) (Renkonen, 2004 #358;Renkonen, 2003 #379), but did not cover the frequent variant *MLH1* c.-93G>A, tested for an allelic loss without sequencing the FLT (Renkonen, 2003 #379), performed short PCRs and subcloning in a second step before sequencing (Renkonen, et al., 2004), or carried out direct sequencing using sequencing primers with variable orientation (Sumitsuji, 2003 #340;Nomura, 2000 #359} Renkonen, 2004 #358). However, most of the cDNA analyses were performed by RT-PCR amplifying only a selected part of the patients' cDNA to specifically investigate splicing of the exon of interest (Auclair, 2006 #371;Baehring, 2006 #269;Borras, 2012 #288;Pagenstecher, 2006 #240). Thereby, alternative/aberrant splicing might be overestimated because shorter isoforms can be preferentially amplified, effects only visible in the FLT amplification such as intergenic inversions might be lost, and different results may be obtained regarding the extent of a SSD due to primer design. As an alternative method, minigene assays examine the inclusion of one specific exon containing a variant in an artificial sequence context in comparison to the wildtype in a monoallelic *ex-vivo* environment using transfected cell lines. This technique has shown a high concordance to patients' cDNA analysis, but may in some cases lead to divergent results regarding the extent and completeness of a SSD, which is depending on the usage of different vectors and types of cell line {Lastella, 2006 #241;Tournier, 2008 #281;van der Klift, 2015 #293}. Due to the absence of the neighbouring genomic context minigene assays can only detect isoforms regarding one exon (Soukarieh, et al., 2016; van der Klift, et al., 2015). This system is laborious and may be oversensitive in some cases especially investigating exons which are alternatively spliced (Lastella, et al., 2006), and is not capable to analyse large exons such as *MSH6* exon 4. Thus, minigene assays are very useful to assess the effect of all variants including intronic variants, when patient RNA is not available, and as a confirmation that a SSD, which was unexpectedly detected in a patients' cDNA, is actually due to a specific variant. Otherwise the FLT cDNA analysis is the preferred method to investigate the effect of a MMR variant upon splicing *in-vivo,* allowing the characterisation of various isoforms that are generated by a SSD, and to test for the transcript integrity. Within the European Mismatch Repair Working Group we agreed on this FLT protocol to be able to compare cDNA results from different laboratories, and by distributing this protocol we aim to achieve a greater analytical sensitivity and data consistency, as it was shown for cDNA analysis of *BRCA1* and *BRCA2* (Whiley, et al., 2014).

### Assessment of normal alternative splicing in controls

In general, the alternative splicing for all MMR transcripts was higher in cDNA+P because oof isoforms were not degraded by NMD. Even though many alternative MMR isoforms are reported (Thompson, et al., 2015), we only recorded those with an intensity of ≥5% in our FLT, and selected against isoforms with an alternative start or end with our method by locating primers in the first and last exon in the wildtype FLT. We detected in each MMR gene at least one alternatively spliced isoform with 5-10% intensity. For *MLH1* we found alternative splicing of exon 1q, 2p, 6, 9, 10, 11, 12, 15, 16, and 17. One isoform lacking exon 1q (r.-62_116del p.?) was new, which may be due to the investigation of the transcript with the longest 5'UTR. In previous *MLH1* FLT analyses, no relevant alternative splicing was described (Etzler, et al., 2008), or specific sequencing primers were used to select against alternatively spliced isoforms (Sumitsuji, et al., 2003). For *MSH2* we recorded alternative splicing of exon 12 and 13, which was also described in partial cDNA analyses (Davoodi-Semiromi, et al., 2000; Mori, et al., 1997; Xia, et al., 1996). In *MSH6* none of the few alternative isoforms reported (Thompson, et al., 2015) showed significant intensity, but we frequently observed skipping of the entire exon 4, which to our knowledge was not described before (Etzler, et al., 2008). This might be due to the fact that this is the first cDNA analysis spanning the huge exon 4 by amplification. For *PMS2* we detected alternative splicing of exon 2p, 4, 4q, 6p, 8, 10, 11, and 14 with significant intensity. Three of those isoforms were novel to our knowledge (exon 8, 11 and 14 separately), and the others were in concordance with a *PMS2* cDNA analysis by two fragments, where alternative splicing of exon 4q and 6p was detected (Etzler, et al., 2008).

The values of alternative splicing exceeded 10% in some exceptions, and reached 15% for *MLH1* exon 1q or *PMS2* exon 6p and 11, and up to 25% for *MSH6* exon 4 (see figure 1), which might either constitute a true physiological condition, or an artefact based on a preferential PCR amplification of the shorter fragment (Vreeswijk and van der Klift, 2012). In previous RT-PCR cDNA studies mostly analysing only parts of the MMR transcripts, the intensity of alternative isoforms was rarely quantified, but described as significantly lower compared to the wildtype (Genuardi, et al., 1998). For *MLH1* and *MSH2* alternative splicing was measured with intensities up to 28% by subcloning (Renkonen, et al., 2004). Higher intensities of alternative splicing in partial cDNA analyses of MMR genes (Charbonnier, et al., 1995; Clarke, et al., 2000; Genuardi, et al., 1998; Plaschke, et al., 1999; Xia, et al., 1996) e.g. ranging from 20-60% for *MLH1* exon 15 and 16 (Palmirotta, et al., 1998) may potentially be overestimated due to a PCR bias for shorter fragments (Vreeswijk and van der Klift, 2012). On the other hand, alternative splicing is reported to vary amongst individuals and tissues {Etzler, 2008 #294;Genuardi, 1998 #20;Palmirotta, 1998 #329}. To sum up, we found several alternative isoforms for *MLH1* and *PMS2,* and one predominant isoform each for *MSH2* and *MSH6.* We set the cut off for alternative splicing up to 10% intensity, which is in concordance with other reports (Plaschke, et al., 1999). For specific exons known to be alternatively spliced (*MLH1* exon 1q, *PMS2* exon 6p and exon 11, *MSH6* exon 4) we tolerate values of- 15%, but postulate that these higher values necessitate further examination regarding allele-specific underrepresentation.

### Biallelic expression and NMD-inhibition

For the analysis of splicing defects frequently resulting in transcripts with an early PTC degraded by NMD (Nagy and Maquat, 1998), a method for reliable NMD-inhibition has to be in place, and was tested in control cohort 2. All MMR transcripts harbouring an early PTC were underrepresented in cDNA-P with intensities of 0-10%. Based on this, we categorized allelic representation ≤10% as an allelic loss. We showed that the early PTC transcripts could be restored in cDNA+P with 15-40% intensity for 80% of the samples, whereas caution is advised for *MSH2.* Despite NMD-inhibition the early PTC variants of *MSH2* in two samples only were detected with 5% or 10% intensity. In literature, the *MSH2* early PTC allele could also be restored to only 12% by NMD-inhibition (Renkonen, et al., 2004), whereas other early PTC variants in *MSH2* were successfully restored by NMD-inhibition (Andreutti-Zaugg, et al., 1997). The underlying reason for this phenomenon could not be determined yet, but as possible explanations may serve the usage of an alternative poly-adenylation signal abrogating amplification with the primers used, mRNA instability, or a mechanistic incomplete NMD-inhibition for *MSH2.*

One has to keep in mind that without an informative variant an allelic loss due to insufficient NMD-inhibition would remain undetected in all non-quantitative cDNA analyses including NGS-based methods. Therefore, our first step in cDNA analyses is to investigate the allelic balance in a heterozygous variant in cDNA+P, which however can be hardly achieved for *MSH2* due to the paucity of SNPs. Biallelic expression is required as a basis for the application of our defined cut off values for aberrant splicing. As expected, samples with late PTC MMR variants were not degraded by NMD. Interestingly, one early PTC in *MLH1* exon 12 and one late PTC in *MSH6* exon 9 generated isoforms skipping the respective exon with intensities of 25-35%, exceeding the normal alternative splicing, and may be regarded as partial SSD. This was also described for other PTC variants and may be relevant for pathogenicity assessment, when a predicted early PTC variant rescues the allele via a SSD resulting in a small *if* deletion (Baehring, et al., 2006; Nystrom-Lahti, et al., 1999; Stella, et al., 2001). Of note, an enhanced alternative splicing was described for samples with pathogenic variants, and its clinical relevance remains uncertain (Auclair, et al., 2006; Thompson, et al., 2015).

### Assessment of aberrant splicing

In order to define the cut off for aberrant splicing for the MMR genes we analysed six samples with variants affecting the conserved splice sites, classified as SSD class 4-5 according to the InSiGHT guidelines. Two splice site variants generated isoforms not subjected to NMD, which were found with 50% intensity in cDNA-P/+P, indicating that the variant abrogated normal splicing and was regarded as complete SSD. For the other variants the aberrantly spliced transcripts leading to an early PTC were strongly degraded by NMD, with < 10% intensity in cDNA-P in three samples. In cDNA+P the NMD-inhibition could restore aberrant isoforms showing intensities of 30-50%. As an exception, one cDNA+P sample showed an intron retention in *MLH1* with only 10-15% intensity, and an allelic imbalance of one allele with 15-20% intensity. Here, either amplification of the larger isoform was less efficient, additional larger isoforms may not be amplified, or NMD-inhibition was insufficient.

For the method implemented here, we set the cut off level for aberrant splicing at ≥30% in cDNA+P for complete SSDs. Partial SSD <30% remain of uncertain clinical significance class 3. In literature, aberrant splicing was defined for partial cDNA analyses by the detection of an isoform intensity within the range of 35-60% (Renkonen, et al., 2004), or 3-fold higher than alternative splicing (Rhine, et al., 2018). However, as we found that NMD-inhibition may restore only 15% of the PTC alleles, also lower intensities of aberrant splicing may need further investigation and have to be interpreted with care, especially when combined with an allelic reduction below 25% intensity (Caux-Moncoutier, et al., 2009; Hesson, et al., 2015; Wei, et al., 1997).

### Cuff off summary:

Taken together, we categorised the allelic representation into biallelic balanced expression (50±10%) or allelic loss (≤10%), and would regard the range between >10% and <40% as unclear with possible allelic reduction, which needs further investigation. We further defined isoforms up to 10% as alternative splicing, and set the cut off for aberrant splicing in cDNA+P to 30-50% intensity for all MMR genes by adding up the intensities of all different isoforms generated by one sequence variant (Figure 3). All splicing intensities between >10% and <30% are of uncertain significance for us and need further analysis. A few exons may show an elevated alternative splicing, such as *MSH6* exon 4, so that in these exons variants may not be properly assessed with these values alone, and investigation of allele-specific splicing helps in pathogenicity assessment.

### Analysis of class 4-5 variants

We investigated five class 5 variants initially predicted as missense variants or in-frame deletions of one amino acid on cDNA level and found aberrant splicing of *MLH1* for three of them affecting exons which are also alternatively spliced.

We found *MLH1* c.793C>T p.(Arg265Cys) as a complete SSD in cDNA affecting not only exon 10 but also neighbouring exons, which is in concordance with a recent cDNA report (LOVD microattribution ID 200068 by Leung) (Soukarieh, et al., 2016), and is supported by the proof of an ESE disruption (Tournier, et al., 2008), whereas only a partial splicing defect skipping exon 10 was described in minigene assays (Tournier, et al., 2008; van der Klift, et al., 2015). Previously, for this variant a decreased protein stability (Perera and Bapat, 2008), and reduced MMR efficiency (Plotz, et al., 2006) were reported.

For the variant *MLH1* c.986A>C p.(His329Pro) classified as pathogenic by segregation (Wang, et al., 1997) we reported a complete SSD, whereas further protein function tests for the predicted missense protein indicated pathogenicity on protein level (Gerykova-Bujalkova, et al., 2008; Raevaara, et al., 2005) or decreased protein stability (Hardt, et al., 2011).

For variant *MLH1* c.1984A>C p.(Thr662Pro) we found a clear but partial SSD with 30% aberrant splicing, which was also described as incomplete SSD for this variant previously in literature (Pagenstecher, et al., 2006), but with no evidence for an ESE disruption (Tournier, et al., 2008).

The two remaining class 5 *MLH1* variants c.1852_1854del p.Lys618del and c.350C>T p.Thr117Met were splice-neutral, and protein instability and MMR impairment have been shown in functional protein assays as the underlying mechanism (Hinrichsen, et al., 2013; Koger, et al., 2018).

In pathogenicity assessment, classification of variants is usually based on data available in public databases, including AF, conservation, splice site prediction and protein modelling. Using these tools, protein predictions revealed a disease-causing effect by three of five algorithms for *MLH1* e17 c.1984A>C p.(Thr662Pro), and five of five for *MLH1* c.793C>T p.(Arg265Cys) without any hint towards a possible SSD. Beside this, for the two variants causing a complete SSD on cDNA level, protein assays indicated defects for the predicted missense proteins never built, so that pathogenicity was wrongly assumed to happen on protein level (Drost, et al., 2010; Plotz, et al., 2006). Therefore, transcript analysis of variants should ideally precede functional protein tests. Beside this functional misclassification, machine learning systems like *in-silico* prediction tools for splicing and protein function are fed with wrong information. *In silico* tools so far seem to recognise the obvious SSD variants as such, but more inconspicuous missense variants generating a SSD cannot be predicted and are wrongly assessed on protein level.

### Method application for VUS and unsolved patients with MMR-deficiency

After method evaluation and threshold definition for biallelic expression/allelic loss and alternative/aberrant splicing we applied our FLT analysis to class 3 variants and unsolved patients suspected of having LS.

Assessment of 21 VUS: Based on our cDNA results, we verified three variants predicted as SSD by the detection of aberrant splicing >30% intensity. One of them showed only a partial SSD described as such previously (Pagenstecher, et al., 2006) and remains class 3, whereas the other two variants induced complete SSDs and allowed reclassification to class 5. The 13 variants predicted to alter a single amino acid (missense and deletion) which were shown to be splice-neutral, remain class 3 as their effect on protein level remains unclear so that further investigation is needed like functional analyses looking at protein stability. Interaction and repair activity do not allow variant classification alone, but can be used in combination with other lines of evidence supporting each other (clinical data, allelic frequency, molecular tumor data, bioinformatical analyses, simulations and predictions) to allow a final classification (Köger 2018).

One genomic *PMS2* exon duplication was shown to alter transcript composition, and even though the duplication cannot be classified by the InSiGHT guidelines because it is *if,* we regard it as class 4 because the protein domain of functional importance for MLH1 interaction is altered.

We designated four synonymous MMR variants as splice-neutral, which now can be re-classified as benign (class 1) according to the InSiGHT criteria.

Within this small number of patients we found 15% of class 3 MMR variants (3/19) to exert a SSD, and these variants were *in-silico* predicted as potential SSD. This number is in concordance with one report (Lastella, et al., 2006), while 25% of all MMR variants in general were reported as SSD (Auclair, et al., 2006). However, the true burden needs to be assessed by much higher sample numbers. Assessment of unsolved patients: Abnormal results in cDNA analyses were obtained for five out of 26 patients presenting as an allelic loss for *PMS2* in one and for *MLH1* in four cases in cDNA-P. NMD-inhibition restored the missing allele in three patients, which were then solved by the detection of genomic pathogenic variants not detected in prior Sanger sequencing. The underlying cause of the allelic loss of *MLH1* in two patients could not be defined in the scope of this work, but the allelic loss was regarded as sufficient proof for a gene defect (LS) potentially based on a regulatory or structural defect. Of note, our method may select against transcripts with large intron retention, duplication of several exons, alternative start or end and fusion transcripts with other genes due to our PCR design, which show up as an allelic loss. Nevertheless we could unambiguously confirm the diagnosis of LS for these patients based on conspicuous cDNA results, which could not be detected by germline sequencing.

RNA-sequencing using next generation sequencing is a new and rapid method to analyse cDNA in a high-throughput scale. However, the advantage of our LR-PCR cDNA analysis is the selection on FLT by amplification followed by a semiquantitative investigation of the splicing pattern and allelic balance by using an informative variant to test transcript integrity. Most of the RT-PCR cDNA analyses published investigated only parts of the MMR transcript by PCR, and may miss an allelic loss or exon deletion/skipping in case of complex splice aberrations or by primer selection (Andreutti-Zaugg, et al., 1997; Borras, et al., 2012; Gerykova-Bujalkova, et al., 2008) (Morak, 2011 #328). In specific constellations, the allelic status of a VUS and a pathogenic variant leading to NMD or a SSD in the same gene can be determined, and used for re-classification if found *in-trans.*

Due to the existence of highly homologous pseudogene sequences (Nicolaides, et al., 1995), the analysis of *PMS2* needs special attention. Our cDNA analysis for the complete *PMS2* transcript prevents a co-amplification of pseudogene transcripts, and allows the verification of pathogenic variants and of exon deletions/duplications, as well as the detection of a gene conversion between *PMS2* and *PMS2CL* (De Vos, et al., 2004; Hayward, et al., 2007).

In regard of the need for precision medicine, the method established here will help to have a higher diagnostic accuracy in several regards: first, in finding regulatory or exonic variants missed by routine sequencing or previous screening. Second, the pathogenicity assessment of class 3 missense or silent variants by cDNA analyses helps to identify SSDs. Third, the correct definition of the mutation mechanism of diverse pathogenic sequence variants like stop mutations, missense variants and small insertions/deletions, which may constitute SSD, will become important in the context of gene therapy. And fourth, the data will help the education of prediction programs by enabling bioinformaticians to work with correct information.

The method can be used in the context of Next generation Sequencing (NGS), e.g. to calibrate high-throughput NGS-based mRNA-assays.

### References

Andersen SD, Liberti SE, Lutzen A, Drost M, Bernstein I, Nilbert M, Dominguez M, Nystrom M, Hansen TV, Christoffersen JW and others. 2012. Functional characterization of MLH1 missense variants identified in Lynch syndrome patients. Hum Mutat 33(12):1647-55.
Andreutti-Zaugg C, Scott RJ, Iggo R. 1997. Inhibition of nonsense-mediated messenger RNA decay in clinical samples facilitates detection of human MSH2 mutations with an in vivo fusion protein assay and conventional techniques. Cancer Res 57(15):3288-93.
Auclair J, Busine MP, Navarro C, Ruano E, Montmain G, Desseigne F, Saurin JC, Lasset C, Bonadona V, Giraud S and others. 2006. Systematic mRNA analysis for the effect of MLH1 and MSH2 missense and silent mutations on aberrant splicing. Hum Mutat 27(2):145-54.
Baehring J, Sutter C, Kadmon M, Doeberitz MV, Gebert J. 2006. A 'nonsense' mutation leads to aberrant splicing of hMLH1 in a German hereditary non-polyposis colorectal cancer family. Fam Cancer 5(2):195-9.
Borras E, Pineda M, Brieger A, Hinrichsen I, Gomez C, Navarro M, Balmana J, Ramon y Cajal T, Torres A, Brunet J and others. 2012. Comprehensive functional assessment of MLH1 variants of unknown significance. Hum Mutat 33(11):1576-88.
Cartegni L, Krainer AR. 2003. Correction of disease-associated exon skipping by synthetic exon-specific activators. Nat Struct Biol 10(2):120-5.
Caux-Moncoutier V, Pages-Berhouet S, Michaux D, Asselain B, Castera L, De Pauw A, Buecher B, Gauthier-Villars M, Stoppa-Lyonnet D, Houdayer C. 2009. Impact of BRCA1 and BRCA2 variants on splicing: clues from an allelic imbalance study. Eur J Hum Genet 17(11):1471-80.
Chan PA, Duraisamy S, Miller PJ, Newell JA, McBride C, Bond JP, Raevaara T, Ollila S, Nystrom M, Grimm AJ and others. 2007. Interpreting missense variants: comparing computational methods in human disease genes CDKN2A, MLH1, MSH2, MECP2, and tyrosinase (TYR). Hum Mutat 28(7):683-93.
Charbonnier F, Martin C, Scotte M, Sibert L, Moreau V, Frebourg T. 1995. Alternative splicing of MLH1 messenger RNA in human normal cells. Cancer Res 55(9):1839-41.
Clarke LA, Jordan P, Boavida MG. 2000. Cell type specificity in alternative splicing of the human mismatch repair gene hMSH2. Eur J Hum Genet 8(5):347-52. Clendenning M, Hampel H, LaJeunesse J, Lindblom A, Lockman J, Nilbert M, Senter L, Sotamaa K, de la Chapelle A. 2006. Long-range PCR facilitates the identification of PMS2-specific mutations. Hum Mutat 27(5):490-5.
Colombo M, Blok MJ, Whiley P, Santamarina M, Gutierrez-Enriquez S, Romero A, Garre P, Becker A, Smith LD, De Vecchi G and others. 2014. Comprehensive annotation of splice junctions supports pervasive alternative splicing at the BRCA1 locus: a report from the ENIGMA consortium. Hum Mol Genet 23(14):3666-80.
Davoodi-Semiromi A, Lanyon GW, Davidson R, Connor MJ. 2000. Aberrant RNA splicing in the hMSH2 gene: molecular identification of three aberrant RNA in Scottish patients with colorectal cancer in the West of Scotland. Am J Med Genet 95(1):49-52.
Davy G, Rousselin A, Goardon N, Castera L, Harter V, Legros A, Muller E, Fouillet R, Brault B, Smirnova AS and others. 2017. Detecting splicing patterns in genes involved in hereditary breast and ovarian cancer. Eur J Hum Genet 25(10):1147-1154.
De Vos M, Hayward BE, Picton S, Sheridan E, Bonthron DT. 2004. Novel PMS2 pseudogenes can conceal recessive mutations causing a distinctive childhood cancer syndrome. Am J Hum Genet 74(5):954-64.
den Dunnen JT, Antonarakis SE. 2000. Mutation nomenclature extensions and suggestions to describe complex mutations: a discussion. Hum Mutat 15(1):7-12.
Drost M, Zonneveld J, van Dijk L, Morreau H, Tops CM, Vasen HF, Wijnen JT, de Wind N. 2010. A cell-free assay for the functional analysis of variants of the mismatch repair protein MLH1. Hum Mutat 31(3):247-53.
Drost M, Zonneveld JB, van Hees S, Rasmussen LJ, Hofstra RM, de Wind N. 2012. A rapid and cell-free assay to test the activity of lynch syndrome-associated MSH2 and MSH6 missense variants. Hum Mutat 33(3):488-94.
Etzler J, Peyrl A, Zatkova A, Schildhaus HU, Ficek A, Merkelbach-Bruse S, Kratz CP, Attarbaschi A, Hainfellner JA, Yao S and others. 2008. RNA-based mutation analysis identifies an unusual MSH6 splicing defect and circumvents PMS2 pseudogene interference. Hum Mutat 29(2):299-305.
Genuardi M, Viel A, Bonora D, Capozzi E, Bellacosa A, Leonardi F, Valle R, Ventura A, Pedroni M, Boiocchi M and others. 1998. Characterization of MLH1 and MSH2 alternative splicing and its relevance to molecular testing of colorectal cancer susceptibility. Hum Genet 102(1):15-20.
Gerykova-Bujalkova M, Krivulcik T, Bartosova Z. 2008. Novel approaches in evaluation of pathogenicity of single-base exonic germline changes involving the mismatch repair genes MLH1 and MSH2 in diagnostics of Lynch syndrome. Neoplasma 55(6):463-71.
Hardt K, Heick SB, Betz B, Goecke T, Yazdanparast H, Kuppers R, Servan K, Steinke V, Rahner N, Morak M and others. 2011. Missense variants in hMLH1 identified in patients from the German HNPCC consortium and functional studies. Fam Cancer 10(2):273-84.
Hayward BE, De Vos M, Valleley EM, Charlton RS, Taylor GR, Sheridan E, Bonthron DT. 2007. Extensive gene conversion at the PMS2 DNA mismatch repair locus. Hum Mutat 28(5):424-30.
Hesson LB, Packham D, Kwok CT, Nunez AC, Ng B, Schmidt C, Fields M, Wong JW, Sloane MA, Ward RL. 2015. Lynch syndrome associated with two MLH1 promoter variants and allelic imbalance of MLH1 expression. Hum Mutat 36(6):622-30.
Hinrichsen I, Brieger A, Trojan J, Zeuzem S, Nilbert M, Plotz G. 2013. Expression defect size among unclassified MLH1 variants determines pathogenicity in Lynch syndrome diagnosis. Clin Cancer Res 19(9):2432-41.
Houdayer C, Caux-Moncoutier V, Krieger S, Barrois M, Bonnet F, Bourdon V, Bronner M, Buisson M, Coulet F, Gaildrat P and others. 2012. Guidelines for splicing analysis in molecular diagnosis derived from a set of 327 combined in silico/in vitro studies on BRCA1 and BRCA2 variants. Hum Mutat 33(8):1228-38. Kayser K, Degenhardt F, Holzapfel S, Horpaopan S, Peters S, Spier I, Morak M, Vangala D, Rahner N, von Knebel-Doeberitz M and others. 2018. Copy number variation analysis and targeted NGS in 77 families with suspected Lynch syndrome reveals novel potential causative genes. Int J Cancer.
Koger N, Paulsen L, Lopez-Kostner F, Della Valle A, Vaccaro CA, Palmero El, Alvarez K, Sarroca C, Neffa F, Kalfayan PG and others. 2018. Evaluation of MLH1 variants of unclear significance. Genes Chromosomes Cancer 57(7):350-358. Kohonen-Corish M, Ross VL, Doe WF, Kool DA, Edkins E, Faragher I, Wijnen J, Khan PM, Macrae F, St John DJ. 1996. RNA-based mutation screening in hereditary nonpolyposis colorectal cancer. Am J Hum Genet 59(4):818-24. Lastella P, Surdo NC, Resta N, Guanti G, Stella A. 2006. In silico and in vivo splicing analysis of MLH1 and MSH2 missense mutations shows exon- and tissue-specific effects. BMC Genomics 7:243.
Li L, Biswas K, Habib LA, Kuznetsov SG, Hamel N, Kirchhoff T, Wong N, Armel S, Chong G, Narod SA and others. 2009. Functional redundancy of exon 12 of BRCA2 revealed by a comprehensive analysis of the c.6853A>G (p.I2285V) variant. Hum Mutat 30(11):1543-50.
Liu Q, Hesson LB, Nunez AC, Packham D, Williams R, Ward RL, Sloane MA. 2016. A cryptic paracentric inversion of MSH2 exons 2-6 causes Lynch syndrome. Carcinogenesis 37(1):10-17.
Lynch HT, de la Chapelle A. 1999. Genetic susceptibility to non-polyposis colorectal cancer. J Med Genet 36(11):801-18.
Mangold E, Pagenstecher C, Friedl W, Mathiak M, Buettner R, Engel C, Loeffler M, Holinski-Feder E, Muller-Koch Y, Keller G and others. 2005. Spectrum and frequencies of mutations in MSH2 and MLH1 identified in 1,721 German families suspected of hereditary nonpolyposis colorectal cancer. Int J Cancer 116(5):692-702.
Mensenkamp AR, Vogelaar IP, van Zelst-Stams WA, Goossens M, Ouchene H, Hendriks-Cornelissen SJ, Kwint MP, Hoogerbrugge N, Nagtegaal ID, Ligtenberg MJ. 2014. Somatic mutations in MLH1 and MSH2 are a frequent cause of mismatch-repair deficiency in Lynch syndrome-like tumors. Gastroenterology 146(3):643-646 e8.
Morak M, Ibisler A, Keller G, Jessen E, Laner A, Gonzales-Fassrainer D, Locher M, Massdorf T, Nissen AM, Benet-Pages A and others. 2018. Comprehensive analysis of the MLH1 promoter region in 480 patients with colorectal cancer and 1150 controls reveals new variants including one with a heritable constitutional MLH1 epimutation. J Med Genet.
Morak M, Koehler U, Schackert HK, Steinke V, Royer-Pokora B, Schulmann K, Kloor M, Hochter W, Weingart J, Keiling C and others. 2011. Biallelic MLH1 SNP cDNA expression or constitutional promoter methylation can hide genomic rearrangements causing Lynch syndrome. J Med Genet 48(8):513-9.
Morak M, Schackert HK, Rahner N, Betz B, Ebert M, Walldorf C, Royer-Pokora B, Schulmann K, von Knebel-Doeberitz M, Dietmaier W and others. 2008. Further evidence for heritability of an epimutation in one of 12 cases with MLH1 promoter methylation in blood cells clinically displaying HNPCC. Eur J Hum Genet 16(7):804-11.
Mori Y, Shiwaku H, Fukushige S, Wakatsuki S, Sato M, Nukiwa T, Horii A. 1997. Alternative splicing of hMSH2 in normal human tissues. Hum Genet 99(5):590-5.
Muller-Koch Y, Kopp R, Lohse P, Baretton G, Stoetzer A, Aust D, Daum J, Kerker B, Gross M, Dietmeier W and others. 2001. Sixteen rare sequence variants of the hMLH1 and hMSH2 genes found in a cohort of 254 suspected HNPCC (hereditary non-polyposis colorectal cancer) patients: mutations or polymorphisms? Eur J Med Res 6(11):473-82.
Nagy E, Maquat LE. 1998. A rule for termination-codon position within intron-containing genes: when nonsense affects RNA abundance. Trends Biochem Sci 23(6):198-9.
Nicolaides NC, Carter KC, Shell BK, Papadopoulos N, Vogelstein B, Kinzler KW. 1995. Genomic organization of the human PMS2 gene family. Genomics 30(2):195-206.
Nystrom-Lahti M, Holmberg M, Fidalgo P, Salovaara R, de la Chapelle A, Jiricny J, Peltomaki P. 1999. Missense and nonsense mutations in codon 659 of MLH1 cause aberrant splicing of messenger RNA in HNPCC kindreds. Genes Chromosomes Cancer 26(4):372-5.
Oliveira C, de Bruin J, Nabais S, Ligtenberg M, Moutinho C, Nagengast FM, Seruca R, van Krieken H, Carneiro F. 2004. Intragenic deletion of CDH1 as the inactivating mechanism of the wild-type allele in an HDGC tumour. Oncogene 23(12):2236-40.
Ollila S, Sarantaus L, Kariola R, Chan P, Hampel H, Holinski-Feder E, Macrae F, Kohonen-Corish M, Gerdes AM, Peltomaki P and others. 2006. Pathogenicity of MSH2 missense mutations is typically associated with impaired repair capability of the mutated protein. Gastroenterology 131 (5):1408-17.
Pagenstecher C, Wehner M, Friedl W, Rahner N, Aretz S, Friedrichs N, Sengteller M, Henn W, Buettner R, Propping P and others. 2006. Aberrant splicing in MLH1 and MSH2 due to exonic and intronic variants. Hum Genet 119(1-2):9-22.
Palmirotta R, Veri MC, Curia MC, Aceto G, D'Amico F, Esposito DL, Arcuri P, Mariani-Costantini R, Messerini L, Mori S and others. 1998. Transcripts with splicings of exons 15 and 16 of the hMLH1 gene in normal lymphocytes: implications in RNA-based mutation screening of hereditary non-polyposis colorectal cancer. Eur J Cancer 34(6):927-30.
Peltomaki P, Vasen H. 2004. Mutations associated with HNPCC predispositionUpdate of ICG-HNPCC/INSiGHT mutation database. Dis Markers 20(4-5):269-76. Perera S, Bapat B. 2008. The MLH1 variants p.Arg265Cys and p.Lys618Ala affect protein stability while p.Leu749Gln affects heterodimer formation. Hum Mutat 29(2):332.
Plaschke J, Bulitta C, Saeger HD, Schackert HK. 1999. Quantitative differences between aberrant transcripts which occur as common isoforms and due to mutation-based exon skipping of the mismatch repair gene hMLH1. Clin Chem Lab Med 37(9):883-7.
Plotz G, Welsch C, Giron-Monzon L, Friedhoff P, Albrecht M, Piiper A, Biondi RM, Lengauer T, Zeuzem S, Raedle J. 2006. Mutations in the MutSalpha interaction interface of MLH1 can abolish DNA mismatch repair. Nucleic Acids Res 34(22):6574-86.
Raevaara TE, Korhonen MK, Lohi H, Hampel H, Lynch E, Lonnqvist KE, Holinski-Feder E, Sutter C, McKinnon W, Duraisamy S and others. 2005. Functional significance and clinical phenotype of nontruncating mismatch repair variants of MLH1. Gastroenterology 129(2):537-49.
Renkonen E, Lohi H, Jarvinen HJ, Mecklin JP, Peltomaki P. 2004. Novel splicing associations of hereditary colon cancer related DNA mismatch repair gene mutations. J Med Genet 41(7):e95.
Renkonen E, Zhang Y, Lohi H, Salovaara R, Abdel-Rahman WM, Nilbert M, Aittomaki K, Jarvinen HJ, Mecklin JP, Lindblom A and others. 2003. Altered expression of MLH1, MSH2, and MSH6 in predisposition to hereditary nonpolyposis colorectal cancer. J Clin Oncol 21(19):3629-37.
Rhine CL, Cygan KJ, Soemedi R, Maguire S, Murray MF, Monaghan SF, Fairbrother WG. 2018. Hereditary cancer genes are highly susceptible to splicing mutations. PLoS Genet 14(3):e1007231.
Santibanez Koref M, Wilson V, Cartwright N, Cunnington MS, Mathers JC, Bishop DT, Curtis A, Dunlop MG, Burn J. 2010. MLH1 Differential allelic expression in mutation carriers and controls. Ann Hum Genet 74(6):479-88.
Soret J, Gabut M, Tazi J. 2006. SR proteins as potential targets for therapy. Prog Mol Subcell Biol 44:65-87.
Soukarieh O, Gaildrat P, Hamieh M, Drouet A, Baert-Desurmont S, Frebourg T, Tosi M, Martins A. 2016. Exonic Splicing Mutations Are More Prevalent than Currently Estimated and Can Be Predicted by Using In Silico Tools. PLoS Genet 12(1):e1005756.
Spurdle AB, Couch FJ, Hogervorst FB, Radice P, Sinilnikova OM, Group IUGVW. 2008. Prediction and assessment of splicing alterations: implications for clinical testing. Hum Mutat 29(11):1304-13.
Steinke V, Engel C, Buttner R, Schackert HK, Schmiegel WH, Propping P. 2013. Hereditary nonpolyposis colorectal cancer (HNPCC)/Lynch syndrome. Dtsch Arztebl Int 110(3):32-8.
Stella A, Wagner A, Shito K, Lipkin SM, Watson P, Guanti G, Lynch HT, Fodde R, Liu B. 2001. A nonsense mutation in MLH1 causes exon skipping in three unrelated HNPCC families. Cancer Res 61(19):7020-4.
Sumitsuji I, Sugano K, Matsui T, Fukayama N, Yamaguchi K, Akasu T, Fujita S, Moriya Y, Yokoyama R, Nomura S and others. 2003. Frequent genomic disorganisation of MLH1 in hereditary non-polyposis colorectal cancer (HNPCC) screened by RT-PCR on puromycin treated samples. J Med Genet 40(3):e30. Takahashi R, Nagai K. 2009. Differences in expression between transcripts using alternative promoters of hMLH1 gene and their correlation with microsatellite instability. Oncol Rep 22(2):265-71.
Thompson BA, Martins A, Spurdle AB. 2015. A review of mismatch repair gene transcripts: issues for interpretation of mRNA splicing assays. Clin Genet 87(2):100-8.
Thompson BA, Spurdle AB, Plazzer JP, Greenblatt MS, Akagi K, Al-Mulla F, Bapat B, Bernstein I, Capella G, den Dunnen JT and others. 2014. Application of a 5-tiered scheme for standardized classification of 2,360 unique mismatch repair gene variants in the InSiGHT locus-specific database. Nat Genet 46(2):107-115. Tournier I, Vezain M, Martins A, Charbonnier F, Baert-Desurmont S, Olschwang S, Wang Q, Buisine MP, Soret J, Tazi J and others. 2008. A large fraction of unclassified variants of the mismatch repair genes MLH1 and MSH2 is associated with splicing defects. Hum Mutat 29(12):1412-24.
Umar A, Boland CR, Terdiman JP, Syngal S, de la Chapelle A, Ruschoff J, Fishel R, Lindor NM, Burgart LJ, Hamelin R and others. 2004. Revised Bethesda Guidelines for hereditary nonpolyposis colorectal cancer (Lynch syndrome) and microsatellite instability. J Natl Cancer Inst 96(4):261-8.
van der Klift HM, Jansen AM, van der Steenstraten N, Bik EC, Tops CM, Devilee P, Wijnen JT. 2015. Splicing analysis for exonic and intronic mismatch repair gene variants associated with Lynch syndrome confirms high concordance between minigene assays and patient RNA analyses. Mol Genet Genomic Med 3(4):327-45.
van der Klift HM, Tops CM, Hes FJ, Devilee P, Wijnen JT. 2012. Insertion of an SVA element, a nonautonomous retrotransposon, in PMS2 intron 7 as a novel cause of Lynch syndrome. Hum Mutat 33(7):1051-5.
Vargas-Parra GM, Gonzalez-Acosta M, Thompson BA, Gomez C, Fernandez A, Damaso E, Pons T, Morak M, Del Valle J, Iglesias S and others. 2017. Elucidating the molecular basis of MSH2-deficient tumors by combined germline and somatic analysis. Int J Cancer 141(7):1365-1380.
Vasen HF, Watson P, Mecklin JP, Lynch HT. 1999. New clinical criteria for hereditary nonpolyposis colorectal cancer (HNPCC, Lynch syndrome) proposed by the International Collaborative group on HNPCC. Gastroenterology 116(6):1453-6. Vreeswijk MP, van der Klift HM. 2012. Analysis and interpretation of RNA splicing alterations in genes involved in genetic disorders. Methods Mol Biol 867:49-63. Wang Y, Friedl W, Lamberti C, Ruelfs C, Kruse R, Propping P. 1997. Hereditary nonpolyposis colorectal cancer: causative role of a germline missense mutation in the hMLH1 gene confirmed by the independent occurrence of the same somatic mutation in tumour tissue. Hum Genet 100(3-4):362-4.
Wei Q, Bondy ML, Mao L, Gaun Y, Cheng L, Cunningham J, Fan Y, Bruner JM, Yung WK, Levin VA and others. 1997. Reduced expression of mismatch repair genes measured by multiplex reverse transcription-polymerase chain reaction in human gliomas. Cancer Res 57(9):1673-7.
Whiley PJ, de la Hoya M, Thomassen M, Becker A, Brandao R, Pedersen IS, Montagna M, Menendez M, Quiles F, Gutierrez-Enriquez S and others. 2014. Comparison of mRNA splicing assay protocols across multiple laboratories: recommendations for best practice in standardized clinical testing. Clin Chem 60(2):341-52.
Xia L, Shen W, Ritacca F, Mitri A, Madlensky L, Berk T, Cohen Z, Gallinger S, Bapat B. 1996. A truncated hMSH2 transcript occurs as a common variant in the population: implications for genetic diagnosis. Cancer Res 56(10):2289-92. Yamaguchi T, Wakatsuki T, Kikuchi M, Horiguchi SI, Akagi K. 2017. The silent mutation MLH1 c.543C>T resulting in aberrant splicing can cause Lynch syndrome: a case report. Jpn J Clin Oncol 47(6):576-580.

## Claims

1. An *in vitro* method for diagnosing Lynch Syndrome, or for determining the pathogenicity of DNA mismatch repair (MMR) gene variants, or for determining the mechanism of pathogenic DNA mismatch repair (MMR) gene defects or Lynch Syndrome, in a patient suffering from Lynch Syndrome, suspected to suffer from Lynch Syndrome or having a risk to develop Lynch Syndrome, the method comprising:
(ai) determining for full-length transcripts (FLTs) of the DNA mismatch repair (MMR) genes *MLH1, MSH2, MSH6* and *PMS2* in a sample comprising cells obtained from the patient:
the sequence of the FLTs, thereby determining the degree of splicing of the exon(s) and the protein sequence encoded by the FLTs, and
(aii) determining allelic balance of a MMR gene of (ai).

2. The method of claim 1, wherein the method further comprises step (b):
(b) diagnosing Lynch Syndrome or determining the pathogenicity of DNA mismatch repair (MMR) gene variants, or determining the mechanism of pathogenic DNA mismatch repair (MMR) gene defects or Lynch Syndrome, in the patient, wherein:
(b1) Lynch Syndrome is diagnosed or a DNA mismatch repair (MMR) gene variant is determined to be pathogenic or likely pathogenic, or not to be splice-neutral
i) in case the allelic balance for a MMR gene is monoallelic with a value of between 0% and 10% (allelic loss), or
ii) in case the allelic balance for a MMR gene is biallelic with a value of between 40% and 60%, and
the degree of splicing is between 30% and 50% for the exons of said MMR gene (aberrant splicing), or
iii) in case the allelic balance for a MMR gene is biallelic with a value of between 40% and 60%, wherein the protein sequence encoded by the FLT of said variant is predicted to be pathogenic or likely pathogenic (InSiGHT class 5 or 4), and
(b2) the patient is determined not to suffer from Lynch syndrome due to a germline MMR gene defect, or a DNA mismatch repair (MMR) gene variant is determined to be splice-neutral, in case the allelic balance for the respective MMR gene is biallelic with a value of between 40% and 60%, wherein: i) the protein sequence encoded by the FLT of said variant is predicted to be benign or likely benign (InSiGHT class 1-2) or ii) said variant is a synonymous variant or intronic variant, and
the degree of splicing is between 0% and 10% for said MMR exons,
with the proviso of exon-specific splice variants of an MMR are tolerated with following values for the degree of splicing between 0% and 15% for skipping of *MLH1* exon 1q, *PMS2* exon 6p and exon 11, and between 0% and 25% for *MSH6* exon 4 (alternative splicing),
or
a DNA mismatch repair (MMR) gene variant is determined to be splice-neutral, in case
the allelic balance for the respective MMR gene is biallelic with a value of between 40% and 60%, and
the degree of splicing is between 0% and 10% for said MMR exons,
with the proviso of exon-specific splice variants of an MMR tolerating following values for the degree of splicing between 0% and 15% for skipping of *MLH1* exon 1q, *PMS2* exon 6p and exon 11, and between 0% and 25% for *MSH6* exon 4 (alternative splicing).

3. The method of claim 1 or 2, wherein in steps (ai) and (aii), the nonsense-mediated mRNA decay (NMD) pathway is active in the cells in said sample.

4. The method of claim 3, wherein step (b) comprises:
(b) diagnosing Lynch Syndrome or determining the pathogenicity of DNA mismatch repair (MMR) gene variants, or determining the mechanism of pathogenic DNA mismatch repair (MMR) gene defects or Lynch Syndrome, in the patient, wherein:
(b1) Lynch Syndrome is diagnosed or a DNA mismatch repair (MMR) gene variant is determined to be pathogenic or likely pathogenic, or not to be splice-neutral,
i) in case the allelic balance for a MMR gene is monoallelic with a value of between 0% and 10%, or
ii) in case the allelic balance for a MMR gene is biallelic with a value of between 40% and 60%, and
the degree of *in-frame* splicing is between 30% and 50% for a MMR gene,
or
iii) in case the allelic balance for a MMR gene is biallelic with a value of between 40% and 60%, wherein the protein sequence encoded by the FLT of said variant is predicted to be pathogenic or likely pathogenic (InSiGHT class 5 or 4), or
(b2) the patient is determined not to suffer from Lynch syndrome due to a germline MMR gene defect, in case
the allelic balance for the MMR gene is biallelic with a value of between 40% and 60%, wherein: i) the protein sequence encoded by the FLT of said variant is predicted to be benign or likely benign (InSiGHT class 1 or 2) or ii) said variant is a synonymous variant or intronic variant, and
the degree of splicing is between 0% and 10% for said MMR exons, with the proviso of exon-specific splice variants of an MMR tolerating following values for the degree of splicing: between 0% and 15% for skipping of *MLH1* exon 1q, *PMS2* exon 6p and exon 11, and between 0% and 25% for *MSH6* exon 4 (alternative splicing),
or
a DNA mismatch repair (MMR) gene variant is determined to be splice-neutral, in case
the allelic balance for the respective MMR gene is biallelic with a value of between 40% and 60%,
and
the degree of splicing is between 0% and 10% for said MMR exons,
with the proviso of exon-specific splice variants of an MMR tolerating following values for the degree of splicing between 0% and 15% for skipping of *MLH1* exon 1q, *PMS2* exon 6p and exon 11, and between 0% and 25% for *MSH6* exon 4 (alternative splicing).

5. The method of claim 2, wherein in steps (ai) and (aii), the nonsense-mediated mRNA decay (NMD) pathway is inhibited in the lymphocytes.

6. The method of claim 5, wherein step (b) comprises:
(b) diagnosing Lynch Syndrome or determining the pathogenicity of DNA mismatch repair (MMR) gene variants, or determining the mechanism of pathogenic DNA mismatch repair (MMR) gene defects or Lynch Syndrome, in the patient, wherein:
(b1) Lynch Syndrome is diagnosed or a DNA mismatch repair (MMR) gene variant is determined to be pathogenic or likely pathogenic, or not to be splice-neutral,
i) in case the allelic balance for a MMR gene is monoallelic with a value of between 0% and 10%, or
ii) in case the allelic balance for a MMR gene is biallelic with a value of between 40% and 60%, and
the degree of splicing is between 30% and 50% for the exons of said MMR gene, or
iii) in case the allelic balance for a MMR gene exhibits allelic reduction with a value of >10% and <40%,
and the degree of splicing is >10% and <30% for the exons of said MMR gene, or
iv) in case the allelic balance for a MMR gene is biallelic with a value of between 40% and 60%, wherein the protein sequence encoded by the FLT of said variant is predicted to be pathogenic or likely pathogenic (InSiGHT class 5 or 4),
(b2) the patient is determined not to suffer from Lynch syndrome due to a germline MMR-defect, in case
the allelic balance for a MMR gene is biallelic with a value of between 40% and 60%, wherein: i) the protein sequence encoded by the FLT of said variant is predicted to be benign or likely benign (InSiGHT class 1 or 2) or ii) said variant is a synonymous variant or intronic variant, and
the degree of splicing is between 0% and 10% for the MMR exons, with the proviso of exon-specific splice variants of an MMR tolerating following values for the degree of splicing: between 0% and 15% for *MLH1* exon 1q, *PMS2* exon 6p, and *PMS2* exon 11, and between 0% and 25% for *MSH6* exon 4,
or
a DNA mismatch repair (MMR) gene variant is determined to be splice-neutral, in case
the allelic balance for the respective MMR gene is biallelic with a value of between 40% and 60%,
and
the degree of splicing is between 0% and 10% for said MMR exons,
with the proviso of exon-specific splice variants of an MMR tolerating following values for the degree of splicing between 0% and 15% for skipping of *MLH1* exon 1q, *PMS2* exon 6p and exon 11, and between 0% and 25% for *MSH6* exon 4 (alternative splicing).

7. The method of any of claims 2 to 6, wherein the method is performed separately with (1) full-length transcripts (FLTs) derived from cells wherein the nonsense-mediated mRNA decay (NMD) pathway is inhibited, and (2) full-length transcripts (FLTs) derived from cells, preferably lymphocytes, wherein the nonsense-mediated mRNA decay (NMD) pathway is active.

8. The method of claim 7, wherein:
(B1) Lynch Syndrome is diagnosed or a DNA mismatch repair (MMR) gene variant is determined to be pathogenic in case the criteria in (b1) of claim 4 are fulfilled and the criteria in (b1) of claim 6 are fulfilled, or
(b2) the patient is determined not to suffer from Lynch syndrome due to a germline MMR gene defect or a DNA mismatch repair (MMR) gene variant is determined to be splice-neutral, in case the criteria in (b2) of claim 4 are fulfilled and the criteria in (b2) of claim 6 are fulfilled.

9. The method of any of claims 1 to 8, wherein diagnosing Lynch Syndrome or determining the pathogenicity of DNA mismatch repair (MMR) gene variants, or determining the mechanism of pathogenic DNA mismatch repair (MMR) gene defects or determining the mechanism of Lynch Syndrome:
comprises identifying a splice site defect (SSD) in at least one full-length transcript (FLT) of an MMR gene, in particular wherein a MMR gene variant is of InSiGHT class 3 (uncertain significance), class 4 (likely pathogenic) or class 5 (pathogenic) with causal connection to the SSD or the SSD is caused by a unknown molecular mechanism/variant, and/or
comprises determining the pathogenicity of a DNA mismatch repair (MMR) gene variant which is a Variant of Uncertain Significance (VUS), and/or
comprises determining the effect of splicing of a DNA mismatch repair (MMR) gene variant of InSiGHT class 3, 4 or 5 on the full-length transcript (FLT), and/or
comprises determining the effect of a germline MMR gene defect by detecting an allelic loss or aberrant splicing in at least one DNA mismatch repair (MMR) gene, in particular with causal connection to a MMR gene variant (of InSiGHT class 3 (uncertain significance), class 4 (likely pathogenic) or class 5 (pathogenic)) or caused by an unknown molecular mechanism/variant.

10. A set of primer pairs suitable for amplifying full-length transcripts (FLTs) or cDNA molecules generated thereof of the DNA mismatch repair (MMR) genes *MLH1, MSH2, MSH6* and *PMS2,*
wherein at least one primer pair is suitable for amplifying full-length transcripts (FLTs) or cDNA molecules generated thereof of *MLH1,* at least one primer pair is suitable for amplifying full-length transcripts (FLTs) or cDNA molecules generated thereof of *MSH2,* at least one primer pair is suitable for amplifying full-length transcripts (FLTs) or cDNA molecules generated thereof of *MSH6* and at least one primer pair is suitable for amplifying full-length transcripts (FLTs) or cDNA molecules generated thereof of *PMS2.*

11. Use of the set of primer pairs of claim 10 for diagnosing Lynch Syndrome, or for determining the pathogenicity of DNA mismatch repair (MMR) gene variants, or for determining the mechanism of pathogenic DNA mismatch repair (MMR) gene defects or Lynch Syndrome, in a patient suffering from Lynch Syndrome, suspected to suffer from Lynch Syndrome or having a risk to develop Lynch Syndrome, and/or for performing a method of any of claims 1-9.

12. The method of any of claims 1 to 9, wherein said sample is a blood sample, in particular whole blood sample, or a tissue sample, in particular sample comprising colon mucosa, sputum, and/or wherein the cells are lymphocytes, in particular Peripheral Blood Lymphocytes (PBL).

13. The method of any of claims 2 to 9, wherein
(i) a variant is characterized as splicing defect when causing aberrant splicing of at least one exon or intron which is located in or in close genomic proximity to the position of said splice variant, and/or
(ii) the patient is determined in step (b2) not to suffer from Lynch syndrome due to a germline MMR gene defect, even though the tumor indicates a MMR-deficiency by an immunohistochemical loss of at least one MMR protein,
(iii) Lynch Syndrome is diagnosed or a DNA mismatch repair (MMR) gene variant is determined to be pathogenic or likely pathogenic, or not to be splice-neutral, in claim 6, alternative (b1)(ii) or (iii), when said splicing is determined to be specific for one allele (allele-specificity).
